# EUROPEAN PATENT SPECIFICATION

(11) **EP 3 773 735 B1**
(45) Date of publication and mention of the grant of the patent: **10.07.2024**
(21) Application number: 19713507.2
(22) Date of filing: 02.04.2019
(51) Int. Cl.: A61K 47/64, A61P 7/00, A61P 35/00

(54) **CONJUGATE OF CYTOTOXIC DRUG AND PRODRUG FORM OF SAID CONJUGATE**
KONJUGAT AUS EINEM ZYTOTOXISCHEN ARZNEIMITTEL UND PRODRUG-FORM AUS DIESEM KONJUGAT
CONJUGUÉ DE MÉDICAMENT CYTOTOXIQUE ET FORME DE PROMÉDICAMENT DUDIT CONJUGUÉ

(30) Priority: 03.04.2018 EP 18305390
(43) Date of publication of application: 17.02.2021
(73) Proprietor: Syndivia, 67083 Strasbourg (FR)
(72) Inventor: KONIEV, Oleksandr, 67200 STRASBOURG (FR); KOLODYCH, Sergii, 67200 STRASBOURG (FR); BONNEFOY, Jean-Yves, 67600 Ebersmunster (FR)
(74) Representative: Cabinet Becker et Associés
(86) International application number: PCT/EP2019/058226
(87) International publication number: WO 2019/192979

(56) References cited:
- US-A1- 2017 095 525
- THIBAUT LEGIGAN ET AL: "Synthesis and Antitumor Efficacy of a .Beta.-Glucuronidase-Responsive Albumin-Binding Prodrug of Doxorubicin", JOURNAL OF MEDICINAL CHEMISTRY,, vol. 55, no. 9, 10 May 2012 (2012-05-10), pages 4516 - 4520, XP002731988, ISSN: 0022-2623, [retrieved on 20120419], DOI: 10.1021/JM300348R

## Description

### FIELD OF THE INVENTION

The present invention relates to a specific conjugate of cytotoxic drugs and prodrug forms of said conjugate, for use in treating cancer and inflammatory diseases.

### TECHNICAL BACKGROUND

Cancer and inflammatory diseases are among the most frequent illnesses nowadays. In particular, cancer is one of the most important cause of death in industrialized countries, and the number of people diagnosed with cancer continues to grow at the current time.

Several treatment modes have been developed so far, however chemotherapy remains the only one that can be used against circulating tumors, such as lymphomas and leukemias, and metastases.

In chemotherapy, one strategy lies in inhibiting polymerization of tubulin and thus preventing cell division (antimitotics). This can be achieved by using compounds of the dolastatin family, in particular, natural dolastatin 10, which can be isolated from the Indian Ocean sea hare *Dolabella auricularia* and consists of four amino-acids, three of which being specific thereto. Synthetic derivatives of dolastatin 10 such as auristatin PE, auristatin E and monomethyl auristatin E (MMAE) also exist and have proven to be efficient inhibitors. The major flaw of such compounds lies in the lack of selectivity towards cancer cells, resulting in the destruction of healthy tissues and therefore intense side effects, such as hair loss or nausea. The development of protein-drug conjugates of such active agents has appeared as an elegant and efficient strategy to overcome the lack of selectivity.

Protein-drug conjugates are by far the fastest growing class of highly potent active pharmaceutical ingredients. Protein-drug conjugate constructs generally involve a protein, such as an antibody, covalently attached to one end of a linker group, on the other end of which is a cytotoxin, i.e. a highly potent cell killing toxin. The protein component of the biomolecule provides target specificity. Once the conjugate enters the cell, the toxin is released, for instance by action of cellular enzymes. Most protein-drug conjugates are indeed directed to cancer treatment. Besides the marketed trastuzumab emtansine (referred to as T-DM1 or Kadcyla^{®}) and the brentuximab vedotin (Adcetris^{®}), a number of protein-drug conjugates are currently undergoing clinical trials for a variety of cancer indications.

One of the main prerequisites for the activity of such conjugates is the efficient release of the cytotoxic drug upon internalization by cancer cells, while the linker between the protein and drug should be stable in blood circulation before the conjugate reaches its biological target.

In this respect, a real breakthrough was achieved by the introduction of enzyme-cleavable linkers into the conjugate construct.

WO 2015/118497 thus describes the β-glucuronidase-responsive albumin-binding conjugate of formula (I), 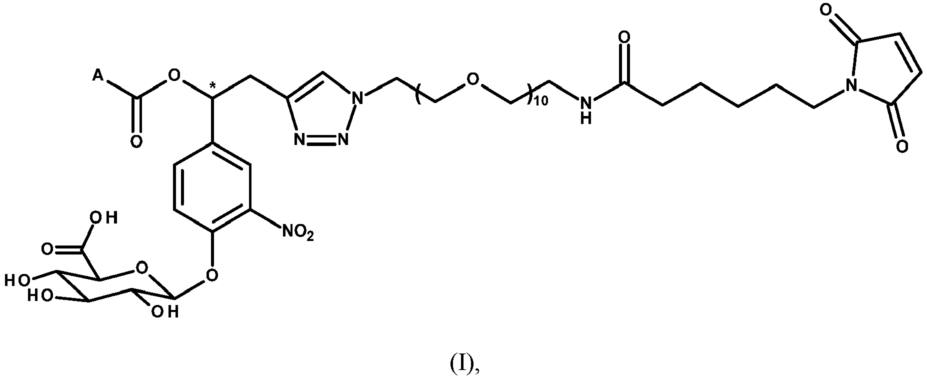 wherein A is typically MMAE. Its maleimide end moiety is intended to provide selective coupling with a circulating albumin after intravenous administration. A glucuronide moiety, which is glucuronidase sensitive, is also present in the molecule and is linked to a self-reactive arm. Since albumin accumulates in tumors and β-glucuronidase is present in the tumor environment, the compound reaches the tumor site where the enzyme catalyzes the cleavage of the glycoside bond and then triggers the release of the drug (MMAE). The conjugate exists as a 1:1 mixture of two isomers, whose varying stereogenic center is on the self-reactive arm (labelled with a star on formula (I)). Legigan et al. (J. Med. Chem 2012, 55, 9, 4516-4520) also describe a racemate of a particular conjugate of formula (I) as represented above, and its use for treating cancer.

### SUMMARY OF THE INVENTION

The inventors demonstrated that the two isomers of the above conjugate, which could not be separated by means of conventional techniques, could be obtained from HPLC enantiomeric separation of an early intermediate. The two isolated isomers were proven stable, especially in human plasma, but the isomer whose varying stereogenic center has an R configuration displayed a higher enzymatic cleavage kinetics than the other one. This higher kinetics observed with one isomer is all the more so unexpected that the stereogenic center is not part of the pharmacophore recognized by the enzyme. This unexpected feature may particularly be beneficial for patients having a lower enzyme expression.

The inventors have also demonstrated that the insertion of a self-immolative moiety into the structure, more specifically between the cytotoxic drug and the remainder of the conjugate, can increase the stability of the conjugate, while preserving its ability to be cleaved (i.e. to release the cytotoxic drug).

Said isomer, which the present invention relates thereto, is a conjugate represented by formula (I): 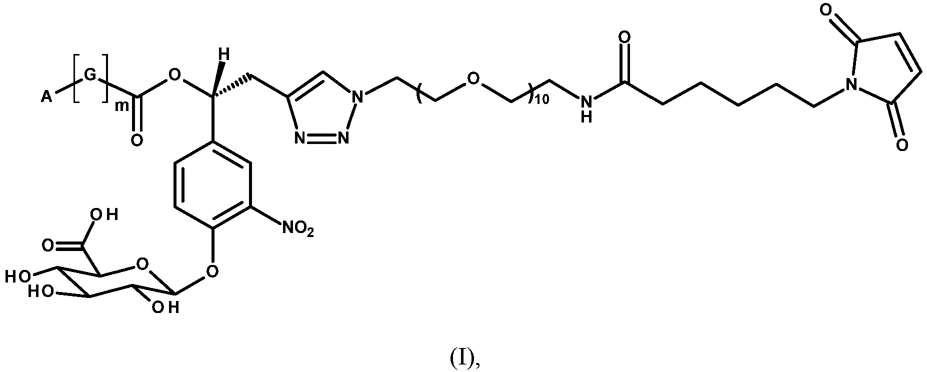 wherein
A represents a radical deriving from a cytotoxic drug,
G is a self-immolative moiety, and
m is 0 or 1,
and pharmaceutically acceptable salts thereof.

The invention further provides a process for preparing a conjugate as defined above, comprising the steps of
a) preparing *rac*-4-(1-hydroxybut-3-yn-1-yl)-2-nitro-phenol from 4-hydroxy-3-nitrobenzaldehyde ;
b) separating and isolating (R)-4-(1-hydroxybut-3-yn-1-yl)-2-nitro-phenol, preferably by chiral high-performance liquid chromatography;
c) reacting (R)-4-(1-hydroxybut-3-yn-1-yl)-2-nitro-phenol with a glucuronic acid derivative, such as methyl acetobromo-α-D-glucuronate, under basic conditions;
d) reacting the compound obtained in step (c) with 4-nitrophenyl chloroformate;
e) coupling the compound obtained in step (d) with a cytotoxic drug or with an A-G-H moiety, in which A represents a radical deriving from a cytotoxic drug and G represents a self-immolative moiety;
f) deprotecting the glucuronide moiety of the compound obtained in step (e), preferably under basic conditions; and
g) coupling the compound obtained in step (f) with an azide of formula N₃-(CH₂-CH₂-O)₁₀-(CH₂)₂-NH-(CO)-(CH₂)₅-X, wherein X is a maleimide group.

The invention also relates to a prodrug comprising at least one molecule of the conjugate as defined above, said molecule of the conjugate being linked via a covalent bond to a protein molecule, preferably albumin, or a fragment thereof,
wherein a "prodrug" refers to at least one conjugate covalently linked to a macromolecule.

It is further directed to a pharmaceutical composition comprising at least an effective amount of at least one conjugate as defined above or at least one prodrug as defined above, and a pharmaceutically acceptable carrier.

This invention still further relates independently to a conjugate as defined above, a prodrug as defined above or a pharmaceutical composition as defined above for use in treating a cancer and/or an inflammatory disease.

### BRIEF DESCRIPTION OF THE DRAWINGS

Figure 1 shows the molecular structure of compound **R1a** from X-ray crystallography. Displacement ellipsoids are drawn at the 50% probability level.
Figure 2 shows the kinetics of β-glucuronidase-induced release of MMAE in plasma solutions of compounds **R5** and **S5.**
Figure 3 shows the kinetics of β-glucuronidase-induced release of MMAF in plasma solutions of compounds **R6** and **S6.**
Figure 4 shows the kinetics of β-glucuronidase-induced release of SN-38 in plasma solutions of compounds **R7** and **S7.**
Figure 5 shows the kinetics of β-glucuronidase-induced release of Doxorubicin in plasma solutions of compounds **R8** and **S8.**
Figure 6 shows the stability of compounds **R5** and **S5** in PBS at pH 7.4.
Figure 7 shows the plasma-binding kinetics of compounds **R5** and **S5.**
Figure 8 shows the plasma stability of albumin-bound **R5** and **S5.**
Figure 9 shows the HPLC profile of the 1: 1 mixture of **R5** and **S5.**
Figure 10 shows the HPLC profile of the 1: 1 mixture of **R4** and **S4.**

### DETAILED DESCRIPTION OF THE INVENTION

The present invention is set out in the appended set of claims.

### Conjugates

The invention relates to a conjugate having the following formula (I): 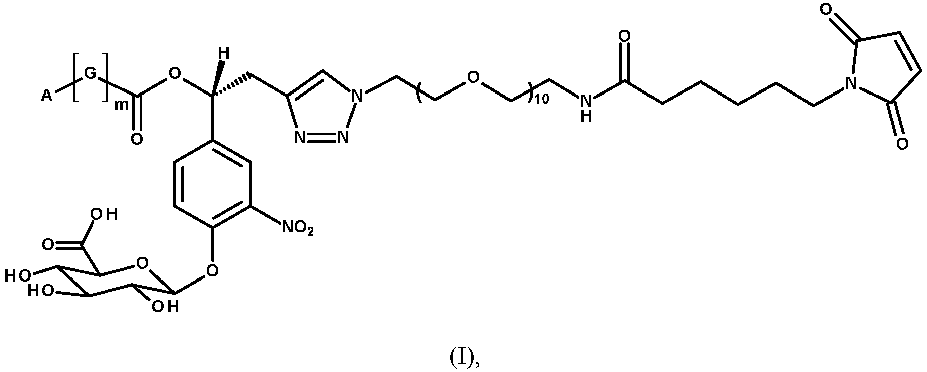 wherein
A represents a radical deriving from a cytotoxic drug,
G is a self-immolative moiety, and
m is 0 or 1,
and pharmaceutically acceptable salts thereof.

In the context of the invention, a "pharmaceutically acceptable salt" refers to a salt of a conjugate or of a prodrug or of a drug according to the invention, and of an alkali metal, of an alkaline-earth metal, or of ammonium, comprising the salts obtained with organic ammonium bases, or salts of a conjugate, or of a prodrug or of a drug according to the invention, and of an organic or inorganic acid.

Salts which are more particularly suitable for the invention may be sodium, potassium, calcium, magnesium salts, quaternary ammonium salts such as tetramethylammonium or tetraethylammonium, and addition salts with ammonia and pharmaceutically acceptable organic amines, such as methylamine, dimethylamine, trimethylamine, ethylamine, triethylamine, ethanolamine or tris(2-hydroxyethyl)amine.

Salts of a conjugate, or of a prodrug or of a drug according to the invention, and of an inorganic acid that are suitable for the invention may be obtained with hydrochloric acid, hydrobromic acid, sulfuric acid, nitric acid or phosphoric acid.

Salts of a conjugate, or of a prodrug or of a drug according to the invention, and of an organic acid that are suitable for the invention may be obtained with carboxylic acids and sulfonic acids, such as formic acid, acetic acid, oxalic acid, citric acid, lactic acid, malic acid, succinic acid, malonic acid, benzoic acid, maleic acid, fumaric acid, tartaric acid, methanesulfonic acid, benzenesulfonic acid or p-toluenesulfonic acid.

According to the invention, a "self-immolative moiety" denotes a divalent chemical group which connects the radical deriving from a cytotoxic drug with the remainder of the conjugate, and which becomes labile upon activation (e.g. enzymatic cleavage of glucuronide residue), leading to a release of the free moieties, in particular the cytotoxic drug. Self immolative moieties are well-known to the skilled artisan (Polym. Chem. 2011, 2, 773-790).

In one embodiment, m is 0.

In another embodiment, m is 1 and G is represented by a formula selected from:

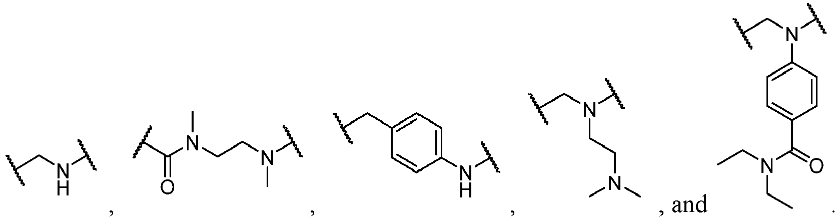

In a preferred embodiment, G is represented by the following formula:

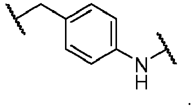

According to the invention, a "radical deriving from a cytotoxic drug" denotes a moiety consisting of a cytotoxic drug deprived of one or more atoms of one of its functional groups (for instance, a H atom) which has reacted with the remainder of the conjugate.

The drug according to the invention is a cytotoxic drug. As used herein, the term "cytotoxic drug" refers to a molecule that when entering in contact with a cell, optionally upon internalization into the cell, alters a cell function (e.g. cell growth and/or proliferation and/or differentiation and/or metabolism such as protein and/or DNA synthesis) in a detrimental way or leads to cell death. As used herein, the term "cytotoxic drug" encompasses toxins, in particular cytotoxins. In principle, a cytotoxic drug is defined as a LO1 ATC molecule ("Anatomical Therapeutic Chemical Classification System" where LO1 is a subgroup defining antineoplastic and immunomodulating agents defined by WHO Collaborating Centre for Drug Statistics Methodology).

The cytotoxic drug according to the invention may be selected from dolastatins such as dolastatin 10, dolastatin 15, auristatin E, auristatin EB (AEB), auristatin EFP (AEFP), monomethyl auristatin F (MMAF), monomethylauristatin-D (MMAD), monomethyl auristatin E (MMAE), 5-benzoylvaleric acid-AE ester (AEVB) or derivatives thereof.

Preferred dolastatins are MMAF, MMAD, MMAE, or derivatives thereof.

More preferred dolastatins are MMAE, MMAF, or derivatives thereof.

In a particular embodiment, the cytotoxic drug according to the invention is an anthracycline such as doxorubicin, idarubicin, epirubicin, daunorubicin, or valrubicin, preferably doxorubicin.

In another particular embodiment, the cytotoxic drug according to the invention is a drug of the camptothecin family (also referred herein to as "camptothecin analog"), such as camptothecin, SN-38, topotecan, irinotecan, exatecan, silatecan, cositecan, lurtotecan, gimatecan, belotecan, or rubitecan, preferably SN-38, exatecan, belotecan, , and more preferably SN-38.

In a preferred embodiment, the cytotoxic drug is selected from doxorubicin, SN-38, MMAE, MMAF, MMAD, and derivatives thereof.

Dolastatins are a family of compounds having a structure of at least 4 amino-acids, preferably 4 amino-acids, at least 3 of which being specific thereto, since different from the 20 amino-acids commonly found naturally. According to the invention, a derivative of dolastatin has a chemical structure very related to at least one compound of the dolastatin family and displays similar antimitotic properties. Structural differences between said derivative and a compound of the dolastatin family may lie in a substitution on at least one side chain of at least one aminoacid by any suitable substituent commonly found. For example, said substituent may be:
- an alkyl group, i.e. a linear or branched saturated hydrocarbon chain, such as a methyl, ethyl, propyl, isopropyl, butyl, sec-butyl, tert-butyl, pentyl, hexyl, heptyl, octyl, nonyl or decyl group;
- a heteroalkyl group, i.e. a linear or branched hydrocarbon chain interrupted by one or more heteroatom(s), mainly O, N and S, such a methoxy or ethoxy group;
- an aryl group, i.e. an aromatic carbocyclic group, such as a phenyl or naphthyl group, which may optionally be substituted with up to four groups including, but not limited to: -COOH, - SO₃H, -OCH₃, -F, -Cl, -Br, -I, -OH, -NH₂, -NO₂, -CN;
- a heteroaryl group, i.e. an aromatic group which contains one or more heteratoms, such as a pyridyl, oxazolyl, furanyl or thiazolyl group; or
- a halogen atom, such as -F, -Cl, -Br, -I.

The structural difference may also consist of a modification of a dolastatin such as dolastatin 10, dolastatin 15, auristatin E, auristatin EB (AEB), auristatin EFP (AEFP), monomethyl auristatin F (MMAF), monomethylauristatin-D (MMAD), monomethyl auristatin E (MMAE), or 5-benzoylvaleric acid-AE ester (AEVB), for example at the level of its tertiary amine in the N-terminal position, so as to render this function compatible with the establishment of a covalent bond with the linker arm under consideration.

The skilled artisan is able to select suitable modifications, in particular suitable substituents, for these purposes.

According to another embodiment, the drug may be selected from mytansins such as DM1 and DM4, anthracyclines such as doxorubicin, nemorubicin and PNU-159682, calicheamicins, duocarymycins such as CC-1065 and duocarmycin A, pyrrolobenzodiazepines, pyrrolobenzodiazepine dimers, indolino-benzodiazepines, indolino-benzodiazepine dimers, α-amanitins, eribulin, acalabrutinib, bleomycin, beritinib, cladribine, clofarabine, cobimetinib, copanlisib, crizotinib, cytarabine, dabrafenib, dactinomycin, daunorubicin, decitabine, epirubicin, ibrutinib, idarubicin, lapatinib, lenalidomide, mitomycin C, mitoxantrone, nelarabine, niraparib, paclitaxel, panobinostat, pomalidomide, prednisone, ribociclib, palbocicilb, rolapitant, rucaparib, sonidegib, tamoxifen, temsirolimus, topotecan, trabectedin, valrubicin, vinblastine, vincristine, and pharmaceutically acceptable salts thereof.

In a particular embodiment, the cytotoxic drug is SN-38, m is 1 and G is

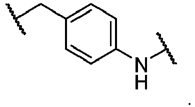

In a another particular embodiment, the cytotoxic drug is a dolastatin or an anthracycline, and m is 0.

### Process

The term "solvent" refers to organic solvent, inorganic solvent such as water, or a mixture thereof. Examples of organic solvents include, but are not limited to, aliphatic hydrocarbons such as pentane or hexane, alicyclic hydrocarbons such as cyclohexane, aromatic hydrocarbons such as benzene, styrene, toluene, *ortho*-xylene, *meta*-xylene or *para*-xylene, halogenated hydrocarbons such as dichloromethane, chloroform or chlorobenzene, nitrogen-based solvents such as pyridine, acetonitrile or triethylamine, oxygen-based solvents, in particular ketones such as acetone, ethers such as diethyl ether, tert-butyl methyl ether (TBME), cyclopentyl methyl ether (CPME), tetrahydrofuran (THF) or methyl tetrahydrofuran (Me-THF), and alcohols such as methanol or ethanol, esters such as n-butyl acetate, or amides such as dimethylformamide (DMF), and mixtures thereof.

"Acid conditions" refers to conditions wherein one or more acids are used. Examples of acid include, but are not limited to, hydrochloric acid, hydrobromic acid, hydriodic acid, hydrofluoric acid, nitric acid, sulfuric acid, hexafluorophosphoric acid, tetrafluoroboric acid, trifluoroacetic acid, acetic acid, sulfonic acid such as methanesulfonic acid, mono- or polycarboxylic acid, or mixtures thereof, preferably hydrochloric acid.

"Basic conditions" refers to conditions wherein one or more bases are used. Examples of base include, but are not limited to, lithium hydroxide or sodium hydroxide, carbonates such as potassium carbonate, sodium carbonate or sodium hydrogenocarbonate, alkoxides such as sodium methoxide, amines such as triethylamine, and nitrogen-based cyclic bases, such as imidazole, N-methylimidazole, pyridine or dimethyl-amino-pyridine (DMAP), preferably lithium hydroxide.

The conjugate according the invention may be prepared from commercial 4-hydroxy-3-nitrobenzaldehyde, through a multi-step synthesis, including a step of enantiomeric separation of the *rac*-4-(1-hydroxybut-3-yn-1-yl)-2-nitro-phenol intermediate by chiral high-performance liquid chromatography (HPLC).

Conditions (temperature, concentration, solvents, reactants, equivalents of the reactants) described below for each step may be adjusted by the skilled artisan using his/her general background in organic synthesis. Each intermediate or product obtained at the end of a step may be isolated and optionally purified, or alternatively, several steps may be carried out one-pot without isolating said intermediate or product. The order of the process steps described below may be modified.

### Step (a)

In one embodiment, *rac*-4-(1-Hydroxybut-3-yn-1-yl)-2-nitro-phenol may be prepared in one step from 4-hydroxy-3-nitrobenzaldehyde. The reaction consists in introducing a propargyl group by addition of the latter on the aldehyde function of 4-hydroxy-3-nitrobenzaldehyde. Typically, said reaction may be performed by reacting 4-hydroxy-3-nitrobenzaldehyde with a nucleophilic propargyl group, i.e. any anionic or organometallic form of the propargyl group, which is able to add on an electrophilic site. Said reaction may be assisted with a catalyst, such as HgCh. Said nucleophilic propargyl group may be formed in situ or beforehand prepared in a separate vessel, from any propargyl source such as propargyl bromide. In a preferred embodiment, the reaction is performed with 4-hydroxy-3-nitrobenzaldehyde and propargyl bromide in the presence of aluminum metal and HgCl₂.

### Step (b)

Then, the two enantiomers of *rac*-4-(1-Hydroxybut-3-yn-1-yl)-2-nitro-phenol may be separated by means of a technique such as chiral HPLC, formation and separation of diastereoisomer salts, crystallization, catalytic resolution or enzymatic resolution, or combinations thereof. Preferably, the two enantiomers are separated by chiral HPLC. Chiral HPLC is a chromatography technique which stationary phase is chiral. The chiral stationary phase may be an amylose-based, cyclodextrin-based or cellulose-based phase. Preferably, the chiral stationary phase is an amylose-based phase.

This step of enantiomeric separation allows to isolate (R)-4-(1-hydroxybut-3-yn-1-yl)-2-nitrophenol. The enantiomeric purity of (R)-4-(1-hydroxybut-3-yn-1-yl)-2-nitro-phenol after the separation step is advantageously more than 90%, preferably more than 95%. Enantiomeric purity may be measured by means of analytical chiral HPLC.

### Step (c)

For the purpose of introducing the glucuronide moiety, (R)-4-(1-hydroxybut-3-yn-1-yl)-2-nitro-phenol may be reacted with a glucuronic acid derivative, under conditions allowing the selective O-glucuronidation of the phenol group. Preferably, said glucuronic acid derivative is a fully-protected glucuronic acid, typically an acetylated glucuronate, and is functionalized so that it can react with the phenol group. Said glucuronic acid derivative is preferably an alkyl acetobromo-α-D-glucuronate, more preferably methyl acetobromo-α-D-glucuronate. In a preferred embodiment, the O-glucuronidation of the phenol group is carried out under basic conditions. For example, the reaction may be carried out in the presence of Ag₂CO₃ and HMTTA (1,1,4,7,10,10-hexamethyltriethylenetetramine).

### Step (d)

The O-glucuronidation product obtained in step (c) may be converted for the purpose of coupling step (e). To this end, the secondary alcohol function of said product may be reacted with a chloroformate, preferably 4-nitrophenyl chloroformate, resulting in the formation of a carbonate. The reaction may be catalyzed by a nucleophile, which may also act as a base, such as pyridine.

### Steps (e), (f) and (g)

In step (e), the compound obtained in step (d) is then linked to a cytotoxic drug as defined above (such as MMAE), or with an A-G-H moiety, in which A represents a radical deriving from a cytotoxic drug and G represents a self-immolative moiety. In a first embodiment, where the cytotoxic drug is directly linked to the compound obtained in step (d), the amine group (or hydroxy group) of said cytotoxic drug reacts with the carbonate of the compound obtained in step (d) to generate an amide group. An activating agent may be used, such as HOAt, HOBt, HOCt. Preferably, said activating agent is HOBt.

Implementation of steps (f) and (g) as described below, subsequently to such a first embodiment, leads to a conjugate of formula (I) as defined above, wherein m is 0.

In a second embodiment, where the cytotoxic drug is linked to the compound obtained in step (d) through a self-immolative moiety, the compound obtained in step (d) is reacted with an A-G-H moiety, in which A represents a radical deriving from a cytotoxic drug and G represents a self-immolative moiety.

A-G-H connects to the compound obtained in step (d) through a functional group, which is advantageously -NH₂ or -NH-.

Examples of A-G-H moieties include, but are not limited to:

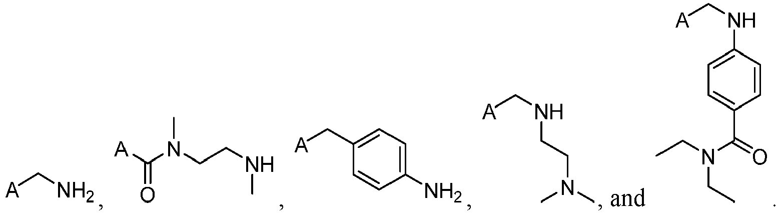

Said A-G-H moiety can typically be prepared by reacting a cytotoxic drug with a compound of formula X-G-H, wherein X represents a leaving group, such as a sulfonate (e.g. methanesulfonate, trifluoromethanesulfonate, toluenesulfonate, nitrobenzenesulfonate), or a halogen (e.g. a chlorine, a bromine, a iodine). Preferably, X is bromine. The cytotoxic drug reacts typically with X-G-H, through its (or one of its) amine or hydroxy group, to form A-G-H as defined above. In a particular embodiment, X-G-H is in a protected form when reacting with the cytotoxic drug. A "protected form" refers to a form wherein one or more reactive functional groups of the compound exist in an unreactive form, such as a NH₂ group protected in the form of a N₃ group. After reacting X-G-H with the cytotoxic drug, A-G-H is obtained and may be in a "protected form" as well. Said A-G-H may be "deprotected" in order to free the reactive functional group(s) (for instance, converting a N₃ group into a NH₂ group).

Implementation of steps (f) and (g) as described below, subsequently to such a second embodiment, leads to a conjugate of formula (I) as defined above, wherein m is 1.

In one embodiment, deprotection of acetyl and ester functions on the glucuronide moiety into hydroxy and carboxylic acid functions, respectively, is then carried out in step (f). Said step (f) of deprotecting the glucuronide moiety of the compound obtained in step (e) is preferably carried out under basic conditions, and more preferably under basic conditions at a low temperature. Alternatively, said step (f) can be carried out under acid conditions, and in any organic solvent, such as acetonitrile. The alkyne function of the compound obtained in step (f) is then coupled with an azide of formula N₃-(CH₂-CH₂-O)₁₀-(CH₂)₂-NH-CO-(CH₂)₅-X, wherein X is a maleimide group, under well-known click-chemistry conditions, allowing the selective formation of the 1,4-disubstituted triazole regioisomer, and thus the formation of a conjugate of the invention.

The azide of formula N₃-(CH₂-CH₂-O)₁₀-(CH₂)₂-NH-CO-(CH₂)₅-X, wherein X is a maleimide group, may be typically obtained by reacting N₃-(CH₂-CH₂-O)₁₀-(CH₂)₂-NH₂ with 2,5-dioxopyrrolidin-1-yl 6-(2,5-dioxo-2,5-dihydro-1H-pyrrol-1-yl)hexanoate.

In another embodiment, step (f) consists in coupling the compound obtained in step (e) with the azide of formula N₃-(CH₂-CH₂-O)₁₀-(CH₂)₂-NH₂ under click-chemistry conditions, allowing the selective formation of the 1,4-disubstituted triazole regioisomer. In step (g), the -NH₂ group is converted to an alkylamide substituted with a maleimide group at the end of the alkyl chain, typically by reacting the compound obtained in step (f) with 2,5-dioxopyrrolidin-1-yl 6-(2,5-dioxo-2,5-dihydro-1H-pyrrol-1-yl)hexanoate. A final deprotection of the glucuronide moiety of the compound obtained in step (g) is then carried out. This deprotection is preferably carried out under basic conditions, and more preferably under basic conditions at a low temperature.

The process according to the invention provides access to a conjugate existing as a single isomer, whose purity is advantageously more than 90%, preferably more than 95%.

### Prodrug

The conjugate according to the invention contains a maleimide group, which is very reactive towards nucleophilic groups, and more specifically towards a thiol group (-SH). Such groups typically add on the maleimide moiety through a Michael addition. The maleimide group is therefore a reactive group of choice for coupling a conjugate of the invention with a macromolecule. In the context of the invention, the macromolecule is preferably an endogenous molecule.

According to the invention, a prodrug refers to at least one conjugate covalently linked to a macromolecule, more specifically a protein. Said prodrug is capable of transporting, in an inactivated form, a cytotoxic drug in an organism, and of releasing said drug into an organ, a tissue or cells, which is (are) specifically targeted, under the action of a β-glucuronidase. The prodrug may be formed *in vivo* or *in vitro* by reacting with a macromolecule, more specifically a protein.

In particular, the protein is selected from those which bind specifically a molecule present at the membrane of a cancer cell, preferably a molecule selected from the group consisting of proteins, glycoproteins, glycolipids, carbohydrates, or a combination thereof, even more preferably the protein binds specifically a protein present at the membrane of a cancer cell. The membrane molecule to which the protein is capable to bind to is a molecule mainly or exclusively present at the membrane of a cancer cell. In a particular embodiment, the membrane molecule recognized by the protein is a protein overexpressed at the membrane of a cancer cell. In a preferred embodiment, the protein may be selected from the group consisting of antibodies and albumins.

As used herein, the terms "antibody" refers to immunoglobulin molecules and immunologically active portions of immunoglobulin molecules, i.e., molecules that contain an antigen-binding site that immunospecifically binds an antigen. As such, the term antibody encompasses not only whole antibody molecules, but also antigen-binding antibody fragments as well as variants of antibodies and antibody fragments. In particular, the antibody according to the invention may correspond to a monoclonal antibody (e.g. a chimeric, humanized or human antibody), or a fragment of monoclonal antibody. The term antibody refers to classical antibodies as well as to heavy-chain antibodies and fragments thereof such as (VHH)2 fragments and single domain antibodies.

Antibody fragments that recognize specific epitopes can be generated by known techniques. The antibody fragments are antigen binding portions of an antibody, such as F(ab')2, Fab, Fv, scFv and the like. Other antibody fragments include, but are not limited to: the F(ab')2 fragments which can be produced by pepsin digestion of the antibody molecule and the Fab' fragments, which can be generated by reducing disulfide bridges of the F(ab')2 fragments. Alternatively, Fab' expression libraries can be constructed (Huse et al., 1989, Science, 246:1274-1281) to allow rapid and easy identification of monoclonal Fab' fragments with the desired specificity.

Antibodies according to the invention may be produced by any technique known in the art, such as, without limitation, any chemical, biological, genetic or enzymatic technique, either alone or in combination. The antibodies of the invention can be obtained by producing and culturing hybridomas. See also WO 2015/063331 for the production of synthetic single domain antibodies.

The antibody according to the invention may be a monomeric antibody or a multimeric antibody and it may comprise at least a variable domain, in particular when the antibody is multimeric. Preferably, the antibody according to the invention is IgG, preferably subtypes IgG1 or IgG4, for example trastuzumab or rituximab.

In a most preferred embodiment of this invention, the protein is an albumin molecule, which has free thiol functions. An endogenous or exogenous albumin, and in particular a human serum albumin, a recombinant albumin or a fragment of an albumin, may be envisaged.

Endogenous albumin is known to accumulate via the "EPR" ("Enhanced Permeability and Retention") effect in the microenvironment of solid tumors, therefore the *in situ* coupling of a conjugate according to the invention with an endogenous albumin molecule makes it possible to target the coupled entity thus formed, also called prodrug, into the tumor microenvironment and thus to overcome the lack of selectivity of the free forms of some cytotoxic drug derivatives, such as dolastatins. It should be noted that such an "EPR" effect applies to the microenvironment of inflamed tissues.

A prodrug may thus be obtained via the selective formation, *in vivo,* of a covalent bond between the conjugate, through its reactive maleimide group, and a free and complementary reactive function, typically a thiol function, of a protein, preferably an endogenous albumin molecule, more preferably a human serum albumin. Said prodrug may comprise at least one molecule of the conjugate according to the invention, covalently linked to a protein molecule or a fragment of the molecule.

In the case of albumin, said covalent bond may be established with the thiol function of the cysteine in position 34 of the albumin.

In a particular embodiment, the prodrug of the invention may be prepared prior to its administration. The prodrug of the invention may be formed *in vitro* through the formation of a covalent bond between at least one conjugate of the invention and a free and complementary function, typically a thiol function, of a protein such as an albumin molecule, a recombinant albumin molecule or a fragment thereof. Said covalent bond may be established with the thiol function of the cysteine in position 34 of the albumin.

In the context of the invention, "fragment of an albumin molecule" denotes a fragment of an albumin molecule having a size sufficient to guarantee satisfactory bioavailability, permeability with respect to tumor tissues and impermeability with respect to the endothelial barrier of healthy tissues, of the prodrug thus generated. In a particular embodiment, the fragment of an albumin molecule contains the cysteine corresponding to the cysteine 34 of the endogenous albumin sequence.

The prodrug according to the invention may be represented by formula (II): 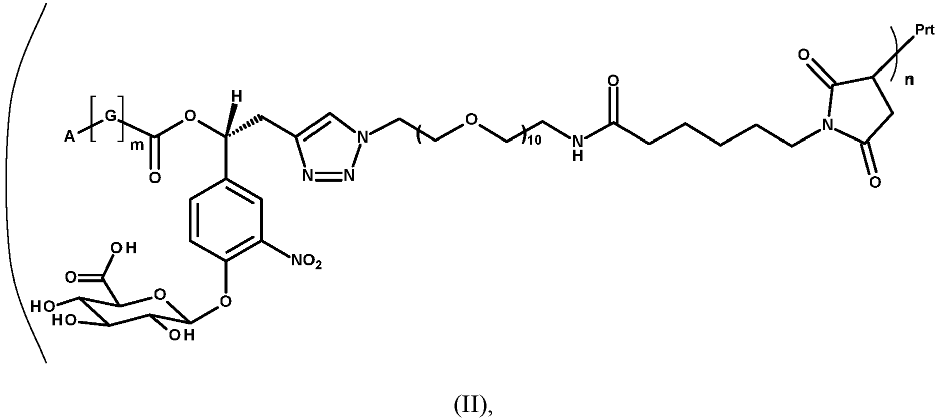
wherein A, G, and m are as defined above,
Prt represents a radical deriving from a protein,
n is comprised between 0.1 and 16, preferably between 0.1 and 8,
and pharmaceutically acceptable salts thereof.

In one particular embodiment, Prt represents a radical deriving from an antibody and n is the mean number of conjugates attached to said antibody and is comprised between 0.1 and 16, preferably between 0.1 and 8.

In another particular embodiment, Prt represents a radical deriving from albumin and n is 1. n may be measured by Mass Spectrometry (MS) and hydrophobic Interaction Chromatography (HIC).

According to the invention, a "radical deriving from a protein" denotes a moiety consisting of a protein deprived of one or more atoms of one of its functional groups which has reacted with the remainder of the conjugate. More specifically, a "radical deriving from a protein" denotes a protein comprising at least one cysteine moiety which has been deprived of the hydrogen atom of its -SH group.

### Pharmaceutical compositions

The conjugates or prodrugs of this invention may be delivered in a pharmaceutical composition comprising at least an effective amount of at least one conjugate of the invention or at least one prodrug of the invention, and a pharmaceutically acceptable carrier. The pharmaceutical composition may be in a solid or liquid state and may be in any of the pharmaceutical forms commonly used in human and/or veterinary medicine, for example in the form of simple or sugar-coated tablets, of pills, of lozenges, of gel capsules, of drops, of granules, of injectable preparations, of ointments, of creams or of gels. The pharmaceutical composition may be prepared according to the usual methods.

As used herein, the term "pharmaceutically acceptable carrier" refers to any ingredient except active ingredients (namely excipients) that is present in a pharmaceutical composition. Its addition may be aimed at conferring a particular consistency or other physical or gustative properties to the final product. An excipient or pharmaceutically acceptable carrier must be devoid of any interaction, in particular chemical, with the actives ingredients. Conventional excipients can be used according to techniques well known by those skilled in the art. As used herein, the term "effective amount" refers to a quantity of an active ingredient, which prevents, removes or reduces the deleterious effects of the disease.

### Uses

The conjugates of the invention or prodrugs of the invention may be used for treating cancer and/or an inflammatory disease.

As used herein, the term "treatment", "treat" or "treating" refers to any act intended to ameliorate the health status of patients such as therapy, prevention, prophylaxis and retardation of the disease. In certain embodiments, such term refers to the amelioration or eradication of a disease or symptoms associated with a disease. In other embodiments, this term refers to minimizing the spread or worsening of the disease resulting from the administration of one or more therapeutic agents to a subject with such a disease.

As used herein, the terms "subject", "individual" or "patient" are interchangeable and refer to an animal, preferably to a mammal, even more preferably to a human. However, the term "subject" can also refer to non-human animals, in particular mammals. The subject according to the invention is an animal, preferably a mammal, even more preferably a human. The subject may be a non-human animal, in particular selected from mammals such as dogs, cats, horses, cows, pigs, sheep and non-human primates, among others. Preferably, the subject is human, preferably an adult, more preferably an adult of at least 40 years old, still more preferably an adult of at least 50 years old, even more preferably an adult of at least 60 years old.

The invention also relates to the use of a conjugate according to the invention, a prodrug according to the invention or a pharmaceutical composition according to the invention, for the preparation of a medicament. Preferably, it relates to a conjugate according to the invention, a prodrug according to the invention or a pharmaceutical composition according to the invention, for the preparation of a medicament for treating cancer and/or an inflammatory disease in a subj ect.

It further relates to a method for treating in a subject a cancer and/or an inflammatory disease, wherein a therapeutically effective amount of a conjugate according to the invention, a therapeutically effective amount of a prodrug according to the invention or a therapeutically effective amount of a pharmaceutical composition according to the invention, is administered to said subject suffering from a cancer and/or an inflammatory disease.

It also relates to a method for treating in a subject a cancer and/or an inflammatory disease, wherein a therapeutically effective amount of a conjugate according to the invention, a therapeutically effective amount of a prodrug according to the invention or a therapeutically effective amount of a pharmaceutical composition according to the invention, is administered to said subject suffering from a cancer and/or an inflammatory disease, in combination with another treatment chosen from a group consisting of chemotherapy, radiotherapy, treatment with at least one anti-inflammatory agent, and combinations thereof.

The conjugate according to the invention, the prodrug according to the invention or the pharmaceutical composition according to the invention may be administered by any convenient route to a subject in need thereof. For instance, it can be administered by a systemic route, in particular by subcutaneous, intramuscular, intravenous or intradermal, preferably by intravenous, injection. The conjugate according to the invention, the prodrug according to the invention or the pharmaceutical composition according to the invention may be administered as a single dose or in multiple doses. The conjugate according to the invention, the prodrug according to the invention or the pharmaceutical composition according to the invention may be administered between every day and every month, preferably every week or every two weeks, more preferably every week. The duration of treatment with a conjugate according to the invention, a prodrug according to the invention or a pharmaceutical composition according to the invention, is preferably comprised between 1 and 20 weeks, preferably between 1 and 10 weeks. Alternatively, the treatment may last as long as the symptoms of the disease persists. The amount of conjugate according to the invention, prodrug according to the invention or pharmaceutical composition according to the invention to be administered has to be determined by standard procedure well known by those of ordinary skills in the art. Physiological data of the patient (e.g. age, size, and weight) and the routes of administration have to be taken into account to determine the appropriate dosage, so as a therapeutically effective amount will be administered to the patient.

### Cancer

The term "cancer" or "tumor", as used herein, refers to the presence of cells possessing characteristics typical of cancer-causing cells, such as uncontrolled proliferation, and/or immortality, and/or metastatic potential, and/or rapid growth and/or proliferation rate, and/or certain characteristic morphological features. This term refers to any type of malignancy (primary or metastases) in any type of subject. It may refer to solid tumor as well as hematopoietic tumor.

Preferably, the cancer according to the invention is selected from the group consisting of the prostate cancer, the lung cancer, the breast cancer, the gastric cancer, the kidney cancer, the ovarian cancer, the hepatocellular cancer, the osteosarcoma, the melanoma, the hypopharynx cancer, the esophageal cancer, the endometrial cancer, the cervical cancer, the pancreatic cancer, the liver cancer, the colon or colorectal cancer, the neuroendocrine tumors, the malignant tumor of the muscle, the adrenal cancer, the thyroid cancer, the uterine cancer, the skin cancer, the bladder cancer, the head and neck cancer, the lymphoma, and the leukemia.

The conjugates of this invention, prodrugs of this invention or pharmaceutical compositions of this invention particularly aim at treating breast, colon, ovarian, prostate, pancreatic and lung cancers.

The conjugates of this invention, prodrugs of this invention or pharmaceutical compositions may be employed, for use thereof in the prevention and/or treatment of metastases.

### Inflammatory diseases

The term "inflammatory disease", as used herein, refers in particular to chronic pathological conditions of the intestine or rheumatoid pathological conditions.

### EXAMPLES

This invention will be better understood in light of the following examples, which are provided for illustrative purposes only.

### Method 1 - preparative HPLC

The preparative HPLC system consisted of two Shimadzu LC-8A pumps, an SPD-10A VP detector (Shimadzu), an SCL-10A VP controller (Shimadzu), an SIL-10A autosampler, a 2 mL sample loop and a SunFire C18 column (150 mm × 19 mm i.d., 5 µm, Waters). The sample was injected into the sample loop and eluted at 17 ml/min flow rate (50 min run, detection at 254 nm; buffer A: H₂O miliQ + 0.05% of TFA; buffer B: acetonitrile; gradient: 40 min - from 5% to 95% B, 5 min - 95% B, 5 min - 5% B).

### Method 2 - analytical HPLC

The analytical HPLC was run on Waters 2695 separation modules equipped with Waters 2487 UV detector and Gemini-NX, 5µm, C18, 150 × 4.6 mm column. The flow rate was 1 ml/min. Solvent A: 0.05% TFA in water. Solvent B: 0.05% TFA in acetonitrile. Gradient run: 0-1 min - 30% B; 1-11 min - 30% to 80% B; 11-12 min - 80 to 95% B; 12-14.5 min - 95% B; 14.5-14.7 min - 95% to 30% B; 14.7-17 min - 30% B.

### Method 3 - LCMS

The LCMS was run on Waters 2695 separation modules equipped with Waters 2487 UV detector, Waters Acquity QDa mass detector and CORTECS, 2.7 µm, C18, 50 × 4.6 mm column. The flow rate was 1 ml/min. Solvent A: 0.05% HCOOH in water. Solvent B: 0.05% HCOOH in acetonitrile. Gradient run: 0-5 min - 5% to 95% B; 5-6 min - 95% B; 6-7.8 min - 5% B. Mass detector was operated in positive MS Scan mode with 600°C probe temperature, 1.5 kV capillary voltage and 10 V cone voltage.

### Method 4 - chiral HPLC

Chiral HPLC was run on Shimadzu SCL-10AVP system equipped with Shimadzu SIL-10A Autosampler, Shimadzu SCL-10AVP UV Detector (set to 254 nm), two Shimadzu LC-8A pumps, CHIRALPAK^{®} IG Semi-Prep Column (5µm, ID 20mm × L 250mm) and a 2 mL sample loop. The flow rate was 20 ml/min. The eluent was dichloromethane. The separation was run for 5 minutes.

### Method 5 - chiral HPLC

Chiral HPLC was run on Shimadzu SCL-10AVP system equipped with Shimadzu SIL-10A Autosampler, Shimadzu SCL-10AVP UV Detector (set to 254 nm), two Shimadzu LC-8A pumps, CHIRALPAK^{®} IG Semi-Prep Column (5µm, ID 20mm × L 250mm) and a 2 mL sample loop. The flow rate was 20 ml/min. The eluent was a mixture of heptane and ethanol (70:30). The separation was run for 25 minutes.

### Synthesis of the racemic mixture of compounds R1 and S1

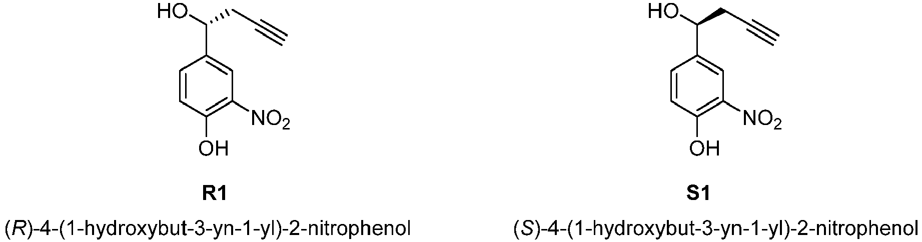

To a flask equipped with a refrigerant and an addition funnel, aluminum powder (6.17 eq., 14156 mg, 524 mmol) and a catalytic quantity of HgCl₂ (0.00248 eq., 57.2 mg, 0.211 mmol) were covered with anhydrous THF (218 mL). Propargyl bromide (6.2 eq., 78439 mg, 58.8 mL, 527 mmol) 80% solution in toluene was then added dropwise (attention: an exothermic reaction; the peak of the exotherm and its intensity depend on the dispersity of aluminum powder). When the addition was over, the resulting reaction mixture was refluxed for 6 hours. The solution was then cooled to 0°C and a solution of 4-hydroxy-3-nitrobenzaldehyde (1 eq., 14210 mg, 85 mmol) in THF (109 mL) was added dropwise. After 30 min of stirring, the aldehyde disappeared completely (controlled by TLC) and the reaction was cooled down to 0°C. The reaction mixture was quenched by a dropwise addition of 1 N HCl (10 mL), and then extracted 3 times with EtOAc. The organic layer was dried over MgSO₄, and then evaporated to yield brown oil, which was purified by flash chromatography (cyclohexane/EtOAc - 70/30). In order to eliminate traces of Wurtz reaction byproduct, the resulting yellow oil was solubilized in DCM (655 mL), the organic layer was extracted 3 times with 1 NNaOH solution (1/3 of DCM volume each time). To the obtained combined aqueous layer was then added DCM (equal volume). The reaction mixture was acidified with concentrated HCl until pH 1. The aqueous phase was extracted 3 more times with DCM (1/3 of the aqueous solution volume each). The combined organic layer was dried over MgSO₄ and evaporated to yield the racemic mixture of (R)-4-(1-hydroxybut-3-yn-1-yl)-2-nitrophenol and (S)-4-(1-hydroxybut-3-yn-1-yl)-2-nitrophenol mixture (7927 mg, 38.3 mmol, 45 %).

**¹H NMR (CHLOROFORM-d)** Shift: 10.63 (s, 1H), 8.23 (s, 1H), 7.73 (dd, J = 8.5, 1.5 Hz, 1H), 7.25 (d, J = 8.5 Hz, 1H), 4.97 (t, J = 6.1 Hz, 1H), 2.67 - 2.97 (m, 3H), 2.20 (t, J = 2.5 Hz, 1H).

**¹³C NMR (CHLOROFORM-d)** Shift: 154.6, 135.2, 135.0, 133.3, 122.3, 120.1, 79.6, 71.9, 70.8, 29.3.

**MS (ESI, Method 3) *m*/*z*:** 230.1 [M+Na]⁺

### Separation of enantiomers R1 and S1

Enantiomers **R1** and **S1** were separated using chiral chromatography. Chiral chromatography was performed on the Shimadzu SCL-10AVP system equipped with Shimadzu SIL-10A Autosampler, Shimadzu SCL-10AVP UV Detector (set to 254 nm), Shimadzu FRC-10A Fraction Collector, two Shimadzu LC-8A pumps, CHIRALPAK^{®} IG Semi-Prep Column (5µm, ID 20mm × L 250mm) and a 2 mL sample loop. Purification was performed at 20 ml/min flow rate using mixture of heptane and ethanol (70:30) as eluent. To 250 mg of the mixture of R1 and S1 were added 0.15 mL of ethanol and 0.35 mL of heptane. The resulting mixture was injected into the sample loop and the purification was run for 25 minutes. A first fraction containing pure R1 isomer was collected between 4 min and 8 min. A second fraction containing pure S1 isomer was collected between 10 min and 22 min. Eluent was evaporated and pure enantiomers R1 and S1 were used in the following steps. Enantiomeric purity of R1 and S1 was confirmed by chiral HPLC (Method 5).

### Compound R1 HPLC (Method 5) RT: 6.53 min

### Compound S1 HPLC (Method 5) RT: 16.79 min

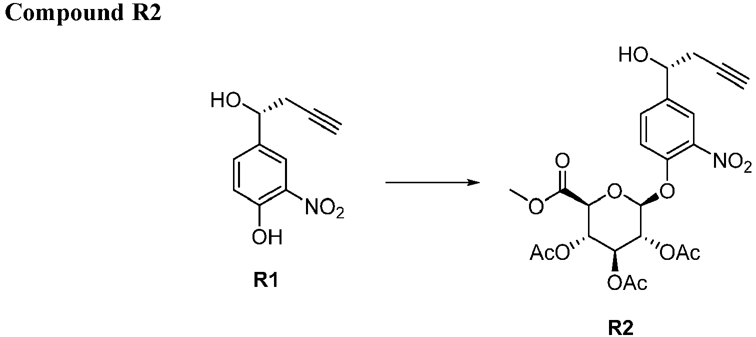

A solution of HMTTA (0.7 eq., 2.11 g, 2.5 mL, 9.17 mmol; 1,1,4,7,10,10-Hexamethyltriethylenetetramine, CAS 3083-10-1) and Ag₂CO₃ (3.7 eq., 13.4 g, 48.5 mmol) in anhydrous acetonitrile (20 mL) was stirred during 2 h at room temperature. (R)-4-(1-hydroxybut-3-yn-1-yl)-2-nitrophenol (1 eq., 2.71 g, 13.1 mmol) and (2R,3R,4S,5S,6S)-2-bromo-6-(methoxycarbonyl)tetrahydro-2H-pyran-3,4,5-triyl triacetate (1.1 eq., 5.7 g, 14.3 mmol) solution in acetonitrile (11.9 mL) was added at 0°C, and the resulting mixture was stirred for 4 h at room temperature. The reaction was quenched with water, followed by the addition of EtOAc (50 mL). The obtained reaction mixture was stirred for 3 minutes, and then filtered to eliminate salts of silver. Extraction with 4 more portions of Et₂O was conducted; the united organic fractions were dried over MgSO₄ and evaporated. The resulting crude material was purified by flash chromatography (cyclohexane/EtOAc gradient 0-100% of EtOAc in 20 minutes; 30 column volumes) to yield methyl **R2** (3428 mg, 6.55 mmol, 50 %) as a pale yellow solid.

**¹H NMR** (CHLOROFORM-d) Shift: 7.86 (dd, J = 6.1, 1.9 Hz, 1H), 7.49 - 7.65 (m, 1H), 7.36 (d, J = 8.5 Hz, 1H), 5.25 - 5.39 (m, 3H), 5.17 - 5.25 (m, 1H), 4.90 (t, J = 6.1 Hz, 1H), 4.21 (d, J = 8.8 Hz, 1H), 3.74 (s, 3H), 2.53 - 2.70 (m, 2H), 1.98 - 2.20 (m, 10H).

**¹³C NMR** (CHLOROFORM-d) Shift: 170.0, 169.3, 169.3, 166.7, 148.5, 141.2, 138.9, 131.2, 122.6, 120.0, 99.8, 90.3, 79.4, 72.6, 72.0, 71.2, 70.7, 70.2, 68.8, 53.0, 29.4, 20.6, 20.5.

**MS (Method** 3) ***m*/*z*:** 546.2 [M+Na]⁺

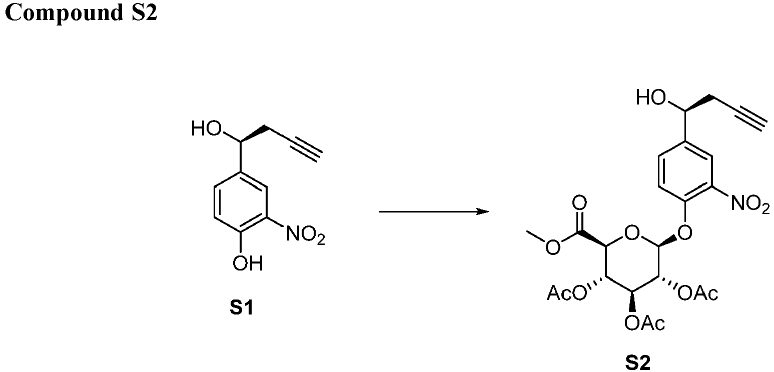

A solution of HMTTA (0.7 eq., 3.4 g, 4 mL, 14.76 mmol; 1,1,4,7,10,10-Hexamethyltriethylenetetramine, CAS 3083-10-1) and Ag₂CO₃ (3.7 eq., 21.5 g, 78 mmol) in anhydrous acetonitrile (30 mL) was stirred during 2 h at room temperature. (S)-4-(1-hydroxybut-3-yn-1-yl)-2-nitrophenol (1 eq., 4.37 g, 21.1 mmol) and methyl (2R,3R,4S,5S,6S)-2-bromo-6-(methoxycarbonyl)tetrahydro-2H-pyran-3,4,5-triyl triacetate (1.1 eq., 10 g, 25.3 mmol) solution in acetonitrile (20 mL) was added at 0°C, and the resulting mixture was stirred for 4 h at room temperature. The reaction was quenched with water, followed by the addition of EtOAc (80 mL). The obtained reaction mixture was stirred for 3 minutes, and then filtered to eliminate salts of silver. Extraction with 4 more portions of Et₂O was conducted; the united organic fractions were dried over MgSO₄ and evaporated. The resulting crude material was purified by flash chromatography (cyclohexane/EtOAc gradient 0-100% of EtOAc in 20 minutes; 30 column volumes) to yield **S2** (6100 mg, 11.6 mmol, 55 %) as a pale yellow solid. **¹H NMR** (CHLOROFORM-d) Shift: 7.86 (dd, J = 6.1, 1.9 Hz, 1H), 7.49 - 7.65 (m, 1H), 7.36 (d, J = 8.5 Hz, 1H), 5.25 - 5.39 (m, 3H), 5.17 - 5.25 (m, 1H), 4.90 (t, J = 6.1 Hz, 1H), 4.21 (d, J = 8.8 Hz, 1H), 3.74 (s, 3H), 2.53 - 2.70 (m, 2H), 1.98 - 2.20 (m, 10H).

**¹³C NMR** (CHLOROFORM-d) Shift: 170.0, 169.3, 169.3, 166.7, 148.5, 141.2, 138.9, 131.2, 122.6, 120.0, 99.8, 90.3, 79.4, 72.6, 72.0, 71.2, 70.7, 70.2, 68.8, 53.0, 29.4, 20.6, 20.5.

**MS (Method 3) *m*/*z*:** 546.3 [M+Na]⁺

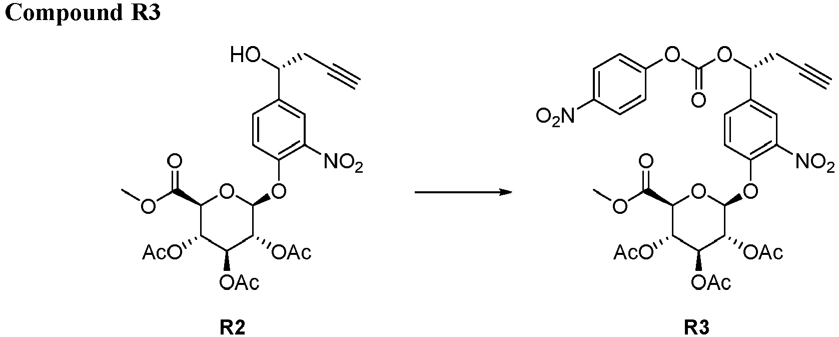

To a solution **of R2** (1 eq., 1600 mg, 3.05 mmol) and 4-nitrophenyl chloroformate (2 eq., 1230 mg, 6.1 mmol) in dry DCM (30 mL) was added pyridine (2.5 eq., 0.62 mL, 7.62 mmol) at 0°C. The mixture was stirred 1 hour at room temperature and then quenched with saturated aqueous NaHCO₃. The mixture was extracted three times with DCM (same as reaction volume) and the combined organic layers were dried over MgSO₄, filtered and concentrated in vacuo. Purification by flash chromatography (cyclohexane/EtOAc gradient 0-100% of EtOAc in 20 minutes; 30 column volumes) afforded **R3** (1469 mg, 2.13 mmol, 70 %) as an off-white solid.

**¹H NMR** (CHLOROFORM-d) Shift: 8.17 - 8.34 (m, 2H), 7.94 (d, J = 2.0 Hz, 1H), 7.65 (dd, J = 8.7, 2.1 Hz, 1H), 7.43 (d, J = 8.5 Hz, 1H), 7.33 - 7.40 (m, 2H), 5.81 (t, J = 6.5 Hz, 2H), 5.21 - 5.43 (m, 4H), 4.25 (d, J = 8.8 Hz, 1H), 3.74 (s, 3H), 2.78 - 3.02 (m, 2H), 2.13 (s, 3H), 2.09 - 2.12 (m, 1H), 2.02 - 2.09 (m, 6H).

**¹³C NMR** (CHLOROFORM-d) Shift: 169.9, 169.2, 169.1, 166.7, 155.2, 151.5, 149.5, 145.6, 141.2, 133.4, 132.1, 125.3, 123.6, 121.7, 119.9, 99.5, 72.7, 72.5, 71.0, 70.2, 68.6, 53.0, 26.3, 20.6, 20.5, 20.5.
**MS (Method 3) *m*/*z*:** 711.2 [M+Na]⁺
**HPLC (Method 4) RT:** 2.77 min

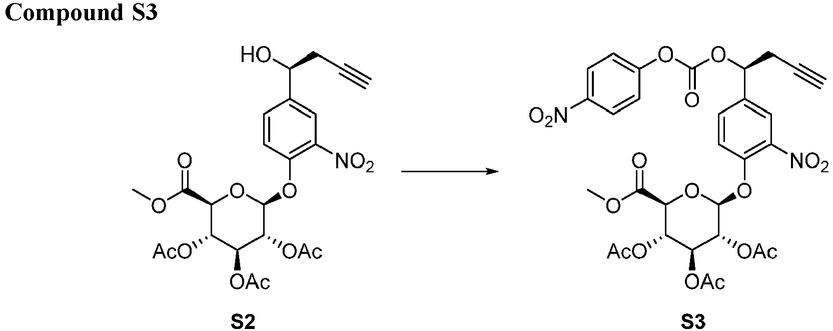

To a solution of **S2** (1 eq., 2600 mg, 5.0 mmol) and 4-nitrophenyl chloroformate (2 eq., 2000 mg, 9.9 mmol) in dry DCM (50 mL) was added pyridine (2.5 eq., 1 mL, 12.42 mmol) at 0°C. The mixture was stirred 1 hour at room temperature, quenched with saturated aqueous NaHCO₃. The mixture was extracted three times with DCM (same as reaction volume) and the combined organic layer was dried over MgSO₄, filtered and concentrated in vacuo. Purification by flash chromatography in cyclohexane/ethyl acetate (cyclohexane/EtOAc gradient 0-100% of EtOAc in 20 minutes; 30 column volumes) yielded **S3** (2300 mg, 2.13 mmol, 68 %) as an off-white solid.

**¹H NMR** (CHLOROFORM-d) Shift: 8.17 - 8.34 (m, 2H), 7.94 (d, J = 2.0 Hz, 1H), 7.65 (dd, J = 8.7, 2.1 Hz, 1H), 7.43 (d, J = 8.5 Hz, 1H), 7.33 - 7.40 (m, 2H), 5.81 (t, J = 6.5 Hz, 2H), 5.21 - 5.43 (m, 4H), 4.25 (d, J = 8.8 Hz, 1H), 3.74 (s, 3H), 2.78 - 3.02 (m, 2H), 2.13 (s, 3H), 2.09 - 2.12 (m, 1H), 2.02 - 2.09 (m, 6H).

**¹³C NMR** (CHLOROFORM-d) Shift: 170.0, 169.3, 169.2, 166.7, 155.2, 151.5, 149.5, 145.6, 141.1, 133.4, 132.1, 125.4, 123.7, 121.7, 119.9, 99.5, 72.6, 72.5, 71.0, 70.2, 68.6, 53.1, 26.2, 20.6, 20.6, 20.5.
**MS (Method 3) *m*/*z*:** 711.2 [M+Na]⁺
**HPLC (Method 4) RT:** 2.84 min

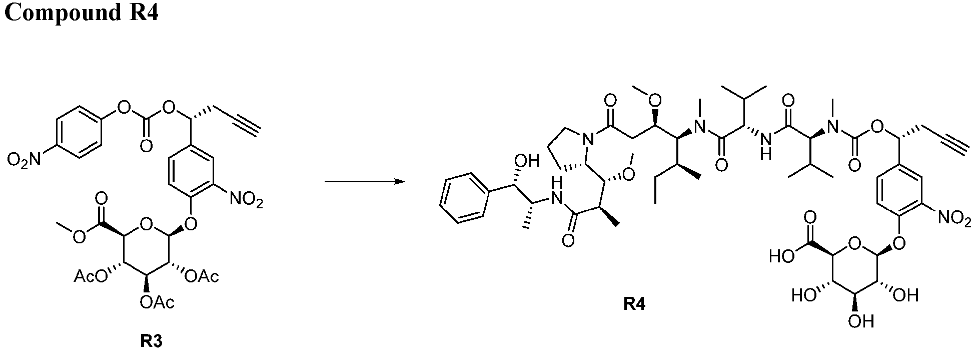

To the solution **of R3** (1 eq., 41.3 mg, 0.06 mmol) and **MMAE** (1 eq., 43.1 mg, 0.06 mmol) in DMF (1.15 mL) was added a solution of HOBt (1 eq., 8.11 mg, 0.06 mmol) in pyridine (0.287 mL). The mixture was stirred at room temperature for 24h then diluted with MeOH (11.5 mL) and cooled to 0°C. To the resulting solution was added LiOH (10 eq., 1 M in H₂O, 0.6 mL, 0.6 mmol) and the mixture was incubated for at 4°C for 16h. The resulting solution was quenched with HCOOH (20 eq., 1 M in H₂O, 1.2 mL, 1.2 mmol) and concentrated to 3 mL under reduced pressure. The residue was purified by preparative HPLC (method 1) to yield **R4** (41.9 mg, 0.0372 mmol, 62 %) as a pale yellow solid.
**HPLC (Method 2) RT:** 7.81 min
**MS (Method 3) *m*/*z*:** 1127.7 [M+H]⁺

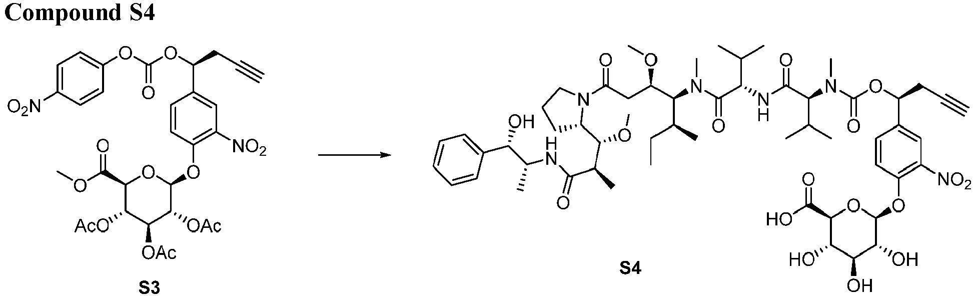

To the solution of **S3** (1 eq., 37.9 mg, 0.055 mmol) and **MMAE** (1 eq., 39.5 mg, 0.055 mmol) in DMF (1.05 mL) was added a solution of HOBt (1 eq., 7.43 mg, 0.055 mmol) in pyridine (0.263 mL). The mixture was stirred at room temperature for 24h then diluted with MeOH (10.5 mL) and cooled to 0°C. To the resulting solution was added LiOH (10 eq., 1 M in H₂O, 0.55 mL, 0.55 mmol) and the mixture was incubated for at 4°C for 16h. The resulting solution was quenched with HCOOH (20 eq., 1 M in H₂O, 1.1 mL, 1.1 mmol) and concentrated to 3 mL under reduced pressure. The residue was purified by preparative HPLC (method 1) to yield **S4** (36.6 mg, 0.0325 mmol, 59 %) as a pale yellow solid.
**HPLC (Method 2) RT:** 7.66 min
**MS (Method 3) *m*/*z*:** 1127.9 [M+H]⁺

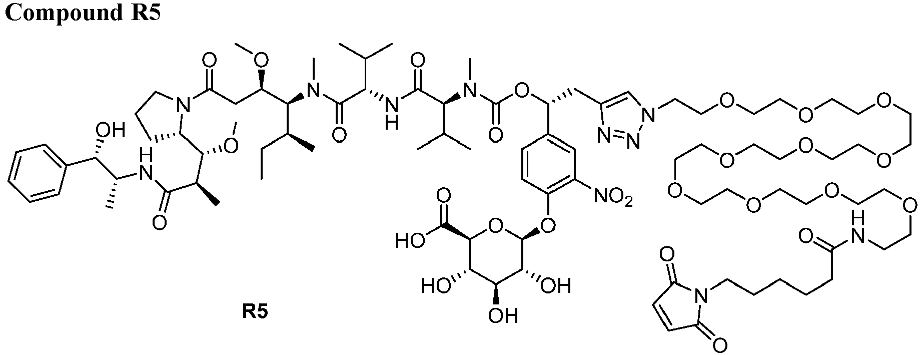

To the solution of 32-azido-3,6,9,12,15,18,21,24,27,30-decaoxadotriacontan-1-amine (1.2 eq., 19 mg, 0.036 mmol) in dry DMF (0.5 mL) was added 2,5-dioxopyrrolidin-1-yl 6-(2,5-dioxo-2,5-dihydro-1H-pyrrol-1-yl)hexanoate (1.2 eq., 11.1 mg, 0.036 mmol) . The mixture was stirred at 21°C for 1 hour and then **R4** (1 eq., 33.8 mg, 0.03 mmol) was added. A TBTA-Cu(II) complex was prepared by mixing tris[(1-benzyl-1H-1,2,3-triazol-4-yl)methyl]amine (0.2 eq., 0.2 M in DMF, 0.03 mL, 0.006 mmol) and copper sulfate pentahydrate (0.2 eq., 0.2 M in H₂O, 0.03 mL, 0.006 mmol) and incubating at 21°C for 2 minutes. The TBTA-Cu(II) complex (0.2 eq., 0.1 M in DMF/H₂O 50/50, 0.06 mL, 0.006 mmol) was added to the reaction mixture followed by sodium ascorbate (1 eq., 1 M in H₂O, 0.03 mL, 0.03 mmol) . The resulting mixture was incubated at 21°C for 1 hour and purified by preparative HPLC to yield **R5** (30.5 mg, 0.0165 mmol, 55 %) as a white solid.
**HPLC (Method 2) RT:** 6.85 min
**MS (Method 3) *m*/*z*:** 924.2 [M+2H]²⁺/2

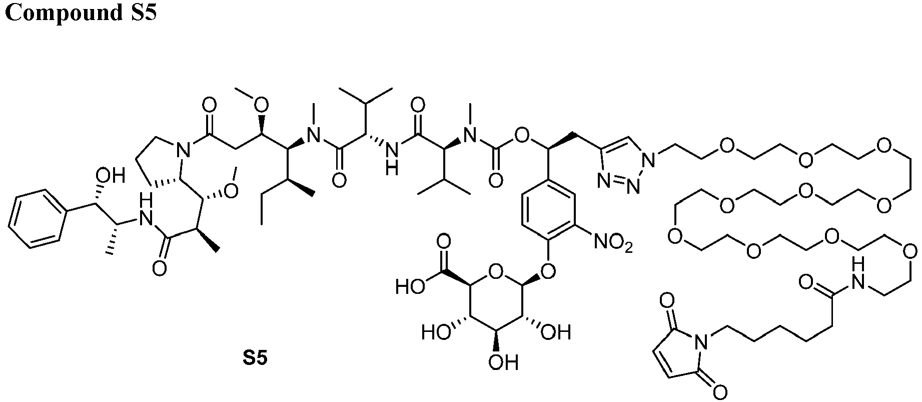

To the solution of 32-azido-3,6,9,12,15,18,21,24,27,30-decaoxadotriacontan-1-amine (1.2 eq., 15.8 mg, 0.03 mmol) in dry DMF (0.417 mL) was added 2,5-dioxopyrrolidin-1-yl 6-(2,5-dioxo-2,5-dihydro-1H-pyrrol-1-yl)hexanoate (1.2 eq., 9.25 mg, 0.03 mmol) . The mixture was stirred at 21°C for 1 hour and then **S4** (1 eq., 28.2 mg, 0.025 mmol) was added. A TBTA-Cu(II) complex was prepared by mixing tris[(1-benzyl-1H-1,2,3-triazol-4-yl)methyl]amine (0.2 eq., 0.2 M in DMF, 0.025 mL, 0.005 mmol) and copper sulfate pentahydrate (0.2 eq., 0.2 M in H₂O, 0.025 mL, 0.005 mmol) and incubating at 21°C for 2 minutes. The TBTA-Cu(II) complex (0.2 eq., 0.1 M in DMF/H₂O 50/50, 0.05 mL, 0.005 mmol) was added to the reaction mixture followed by sodium ascorbate (1 eq., 1 M, 0.025 mL, 0.025 mmol) . The resulting mixture was incubated at 21°C for 1 hour and purified by preparative HPLC to yield **S5** (26.8 mg, 0.0145 mmol, 58 %) as a white solid.
**HPLC (Method 2) RT:** 6.65 min
**MS (Method 3) *m*/*z*:** 924.1 [M+2H]²⁺/2

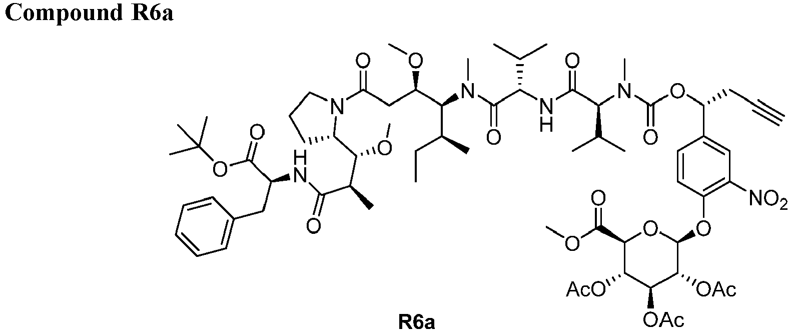

To the solution of **R3** (1 eq., 41.5 mg, 0.06 mmol) and **MMAF** (1 eq., 47.5 mg, 0.06 mmol) in DMF (1.3 mL) was added a solution of HOBt (1 eq., 8.14 mg, 0.06 mmol) in pyridine (0.291 mL). The mixture was stirred at room temperature for 66h, quenched with HCOOH (3 eq., 6.8 µL, 0.18 mmol) and purified by preparative HPLC (Method 1) to afford **R6a** (57 mg, 0.043 mmol, 71% yield) as a white solid.
**HPLC (Method 2) RT:** 6.0 min
**MS (Method 3) *m*/*z*:** 669.5 [M+2H]²⁺/2

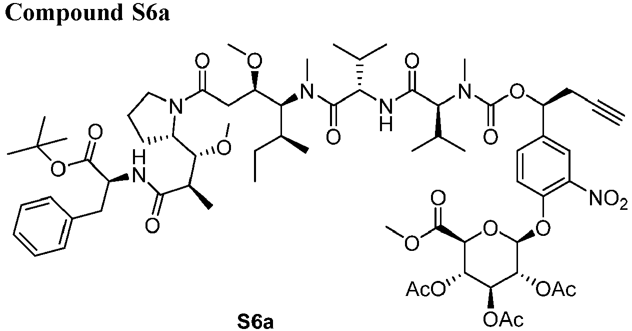

To the solution of **S3** (1 eq., 41.5 mg, 0.06 mmol) and **MMAF** (1 eq., 47.5 mg, 0.06 mmol) in DMF (1.3 mL) was added a solution of HOBt (1 eq., 8.14 mg, 0.06 mmol) in pyridine (0.291 mL). The mixture was stirred at room temperature for 66h, quenched with HCOOH (3 eq., 6.8 µL, 0.18 mmol) and purified by preparative HPLC (method 1) to afford **S6a** (26.6 mg, 0.02 mmol, 33% yield) as a white solid.
**HPLC (Method 2) RT:** 6.0 min
**MS (Method 3) *m*/*z*:** 669.4 [M+2H]²⁺/2

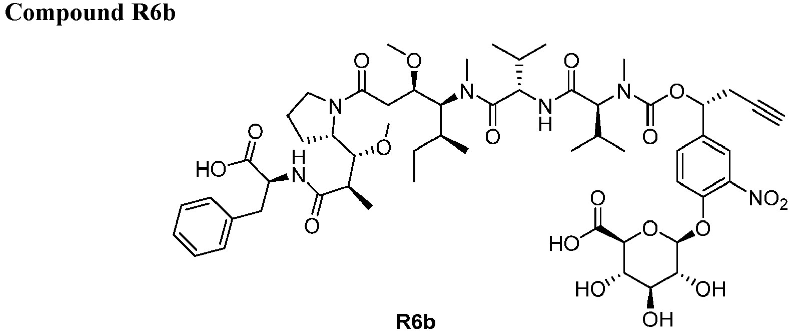

To the solution of **R6a** (1 eq. 26.5 mg, 0.02 mmol) in MeCN (233 µL) was added concentrated aqueous HCl (233 µL) and the solution was stirred at 40°C for 20h. The mixture was then diluted with 0.5 mL of MeCN and centrifuged to separate the precipitate. The supernatant was purified by preparative HPLC (method 1) to afford **R6b** (6.7 mg, 5.9 µmol, 30% yield) as an off-white solid.
**HPLC (Method 2) RT:** 4.4 min
**MS (Method 3) *m*/*z*:** 1141.6 [M+H]⁺

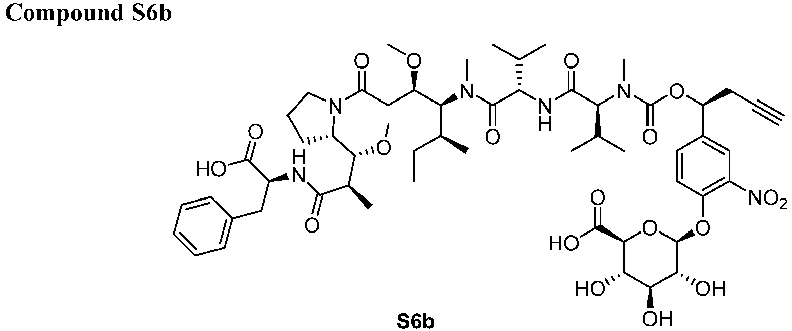

To the solution of **S6a** (1 eq. 26.5 mg, 0.02 mmol) in MeCN (233 µL) was added concentrated aqueous HCl (233 µL) and the solution was stirred at 40°C for 20h. The mixture was then diluted with 0.5 mL of MeCN and centrifuged to separate the precipitate. The supernatant was purified by preparative HPLC (method 1) to afford **S6b** (10.4 mg, 9.1 µmol, 46% yield) as an off-white solid.
**HPLC (Method 2) RT:** 4.4 min
**MS (Method 3) *m*/*z*:** 1141.6 [M+H]⁺

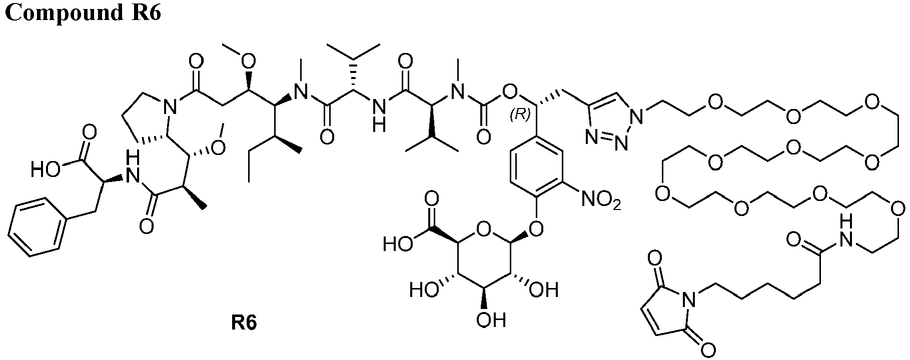

To the solution of 32-azido-3,6,9,12,15,18,21,24,27,30-decaoxadotriacontan-1-amine (1.2 eq., 3.53 mg, 6.7 µmol) in dry DMF (0.15 mL) was added a solution of 2,5-dioxopyrrolidin-1-yl 6-(2,5-dioxo-2,5-dihydro-1H-pyrrol-1-yl)hexanoate (1.2 eq., 2.07 mg, 6.7 µmol) in dry DMF (0.15 mL). The mixture was stirred at 21°C for 1 hour and then **R6b** (1 eq., 6.4 mg, 5.6 µmol) in DMSO (0.15 mL) was added. A THPTA-Cu(II) complex was prepared by mixing tris(3-hydroxypropyltriazolylmethyl)amine (1.5 eq., 0.1 M in DMF, 0.084 mL, 8.4 µmol) and copper sulfate pentahydrate (1.5 eq., 0.1 M in H₂O, 0.084 mL, 8.4 µmol) and incubating at 21°C for 1 minute. The THPTA-Cu(II) complex (1.5 eq., 0.05 MinDMF/H₂O 50/50, 0.168 mL, 8.4 µmol) was added to the reaction mixture followed by sodium ascorbate (7.46 eq., 0.5 M in H₂O, 0.084 mL, 41.5 µmol). The resulting mixture was incubated at 21°C for 15 min and purified by preparative HPLC (method 1) to yield **R6** (3.7 mg, 1.99 µmol, 36 %) as a white solid.
**HPLC (Method 2) RT:** 4.18 min
**MS (Method 3) *m*/*z*:** 931.1 [M+2H]²⁺/2

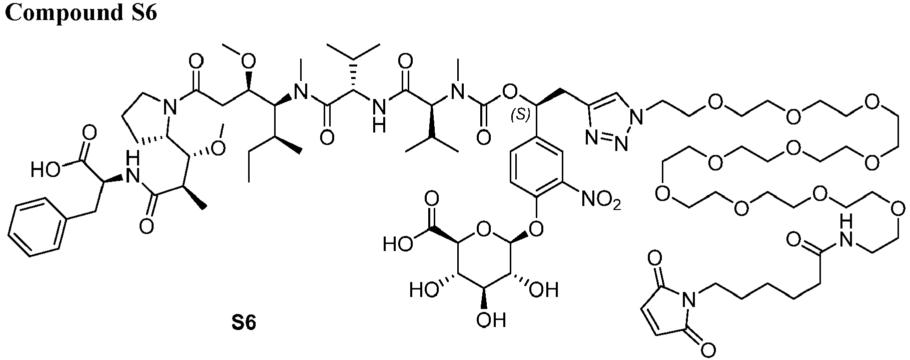

To the solution of 32-azido-3,6,9,12,15,18,21,24,27,30-decaoxadotriacontan-1-amine (1.2 eq., 4.63 mg, 8.8 µmol) in dry DMF (0.15 mL) was added a solution of 2,5-dioxopyrrolidin-1-yl 6-(2,5-dioxo-2,5-dihydro-1H-pyrrol-1-yl)hexanoate (1.2 eq., 2.71 mg, 8.8 µmol) in dry DMF (0.15 mL). The mixture was stirred at 21°C for 1 hour and then **S6b** (1 eq., 8.3 mg, 7.3 µmol) in DMSO (0.15 mL) was added. A THPTA-Cu(II) complex was prepared by mixing tris(3-hydroxypropyltriazolylmethyl)amine (1.5 eq., 0.1 M in DMF, 0.109 mL, 10.9 µmol) and copper sulfate pentahydrate (1.5 eq., 0.1 M in H₂O, 0.109 mL, 10.9 µmol) and incubating at 21°C for 1 minute. The THPTA-Cu(II) complex (1.5 eq., 0.05 M in DMF/H₂O 50/50, 0.218 mL, 10.9 µmol) was added to the reaction mixture followed by sodium ascorbate (7.46 eq., 0.5 M in H₂O, 0.109 mL, 54.4 µmol). The resulting mixture was incubated at 21°C for 15 min and purified by preparative HPLC (method 1) to yield **S6** (6.2 mg, 3.33 µmol, 46 %) as a white solid.
**HPLC (Method 2) RT:** 4.14 min
**MS (Method 3) *m*/*z*:** 931.1 [M+2H]²⁺/2

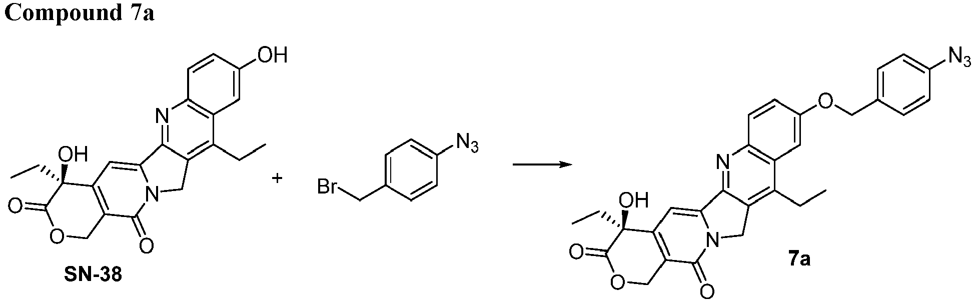

To a solution of SN-38 (1 eq., 72.2 mg, 0.18 mmol) and 1-azido-4-(bromomethyl)benzene (1 eq., 39 mg, 0.18 mmol) in DMF (7.22 mL) was added K₂CO₃ (3 eq., 76.3 mg, 0.55 mmol). The resulting solution was purged with nitrogen, and stirred for 1h at room temperature to allow the formation of **7a.** Crude **7a** (92 mg, 0.18 mmol, 96%) was used in the next step without further purification.

**MS (Method 3) *m*/*z*:** 524.2 [M+H]⁺

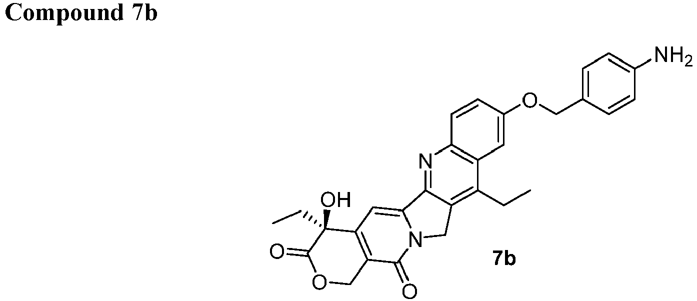

To a solution of **7a** (1 eq., 96 mg, 0.18 mmol) in DMF (3mL) was added tris(2-carboxyethyl)phosphine hydrochloride (TCEP, 1.3 eq., 68.33 mg, 0.24 mmol), followed by the addition of K₂CO₃ (3 eq., 76 mg, 0.55 mmol). The mixture was stirred for 1 hour, then 5x volume of EtOAc was added, followed by 1x volume of water. Organic layer was separated, and aqueous layer was extracted with another 5x volume of EtOAc. Combined organic layer was dried over MgSO₄ and concentrated under vacuum to yield **7b** (90 mg, 0.18 mmol, 99%) as a pale yellow solid. Crude **7b** was used in the next step without further purification.

**MS (Method 3) *m*/*z*:** 498.2 [M+H]⁺

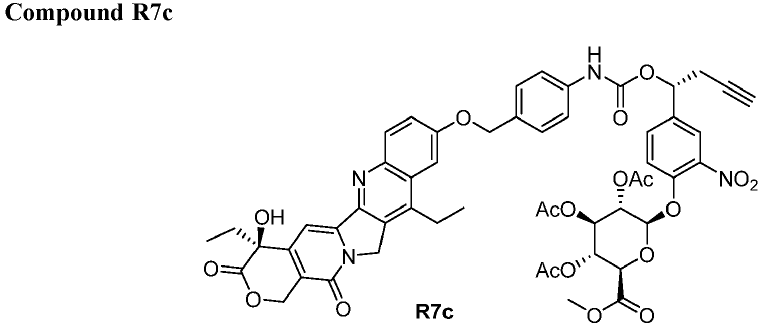

A solution **of R3** (1 eq., 82 mg, 0.12 mmol), **7b** (1 eq., 59 mg, 0.12 mmol) and HOBt (1 eq., 16 mg, 0.12 mmol) in DMF (2.4 mL) was stirred at 25°C for 16 hours. The resulting mixture was purified by preparative HPLC (method 1) to yield **R7c** (45 mg, 0.043 mmol, 36%) as a pale yellow solid.

**MS (Method 3) *m*/*z*:** 1047.3 [M+H]⁺

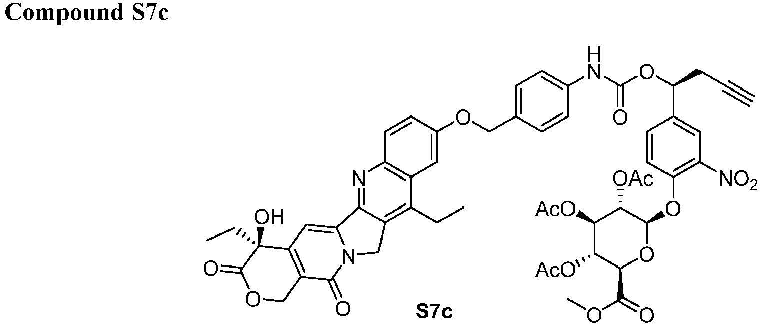

A solution of **S3** (1 eq., 82 mg, 0.12 mmol), **7b** (1 eq., 59 mg, 0.12 mmol) and HOBt (1 eq., 16 mg, 0.12 mmol) in DMF (2.4 mL) was stirred at 25°C for 16 hours. The resulting mixture was purified by preparative HPLC (method 1) to yield **S7c** (63 mg, 0.06 mmol, 50%) as a pale yellow solid.

**MS (Method 3) *m*/*z*:** 1047.3 [M+H]⁺

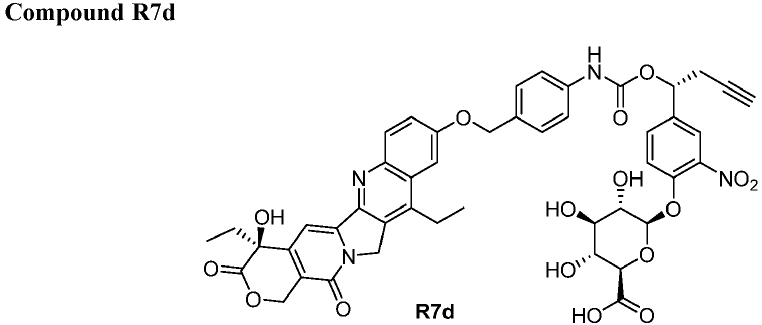

To a solution **of R7c** (1 eq., 23 mg, 22 µmol) in MeOH (20 mL) was added 1M aqueous solution of LiOH (200 eq., 4.4 mL, 4.4 mmol). The resulting reaction mixture was stirred at 25°C for 4h and purified by preparative HPLC to yield **R7d** (16 mg, 0.018 mmol, 80%) as a pale yellow solid.

**MS (Method 3) *m*/*z*:** 907.3 [M+H]⁺

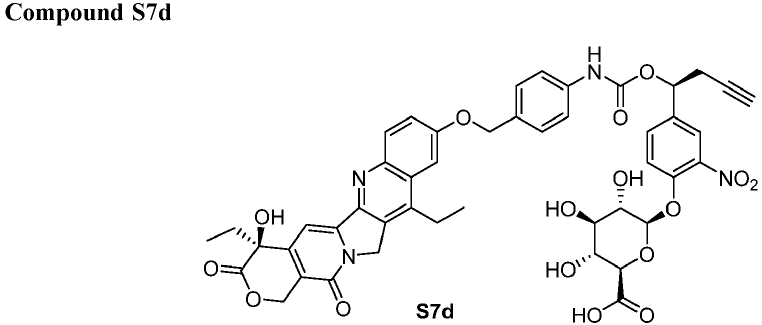

To a solution of **S7c** (1 eq., 23 mg, 22 µmol) in MeOH (20 mL) was added 1M aqueous solution of LiOH (200 eq., 4.4 mL, 4.4 mmol). The resulting reaction mixture was stirred at 25°C for 4h and purified by preparative HPLC to yield **S7d** (13.9 mg, 0.016 mmol, 69.6%) as a pale yellow solid.

**MS (Method 3) *m*/*z*:** 907.3 [M+H]⁺

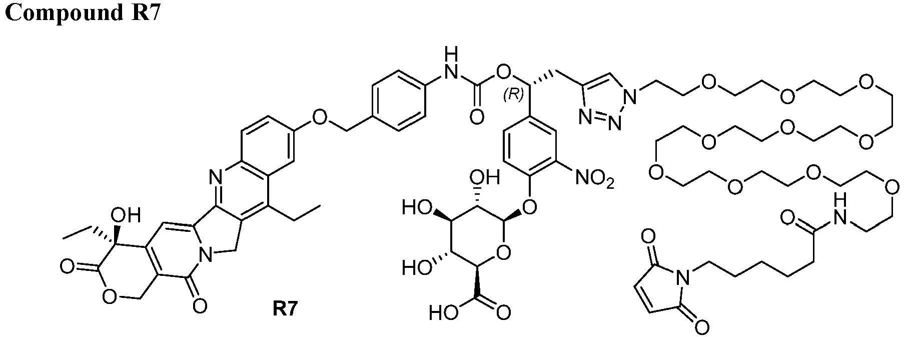

To the solution of 32-azido-3,6,9,12,15,18,21,24,27,30-decaoxadotriacontan-1-amine (1.5 eq., 5.92 mg, 11.3 µmol) in dry DMF (0.1 mL) was added a solution of 2,5-dioxopyrrolidin-1-yl 6-(2,5-dioxo-2,5-dihydro-1H-pyrrol-1-yl)hexanoate (1.5 eq., 3.47 mg, 11.3 µmol) in dry DMF (0.1 mL). The mixture was stirred at 21°C for 1 hour and then **R7d** (1 eq., 6.8 mg, 7.5 µmol) in DMSO (0.1 mL) was added. A THPTA-Cu(II) complex was prepared by mixing tris(3-hydroxypropyltriazolylmethyl)amine (0.5 eq., 0.1 M in DMF, 37.5 µL, 3.75 µmol) and copper sulfate pentahydrate (0.5 eq., 0.1 M in H₂O, 37.5 µL, 3.75 µmol) and incubating at 21°C for 1 minute. The THPTA-Cu(II) complex (0.5 eq., 0.05 M in DMF/H₂O 50/50, 75 µL, 3.75 µmol) was added to the reaction mixture followed by sodium ascorbate (2.5 eq., 0.5 M in H₂O, 37.5 µL, 18.75 µmol). The resulting mixture was incubated at 21°C for 15 min and purified by preparative HPLC (method 1) to yield **R7** (6.95 mg, 4.28 µmol, 57 %) as a white solid.
**HPLC (Method 2) RT:** 3.92 min
**MS (Method 3) *m*/*z*:** 813.9 [M+2H]²⁺/2

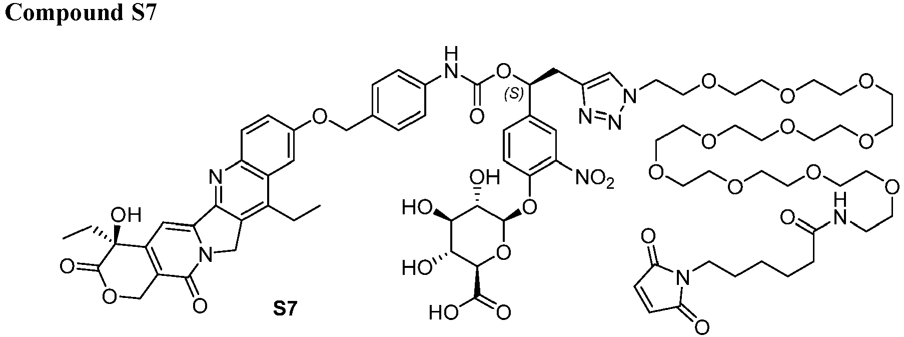

To the solution of 32-azido-3,6,9,12,15,18,21,24,27,30-decaoxadotriacontan-1-amine (1.5 eq., 5.92 mg, 11.3 µmol) in dry DMF (0.1 mL) was added a solution of 2,5-dioxopyrrolidin-1-yl 6-(2,5-dioxo-2,5-dihydro-1H-pyrrol-1-yl)hexanoate (1.5 eq., 3.47 mg, 11.3 µmol) in dry DMF (0.1 mL). The mixture was stirred at 21°C for 1 hour and then **S7d** (1 eq., 6.8 mg, 7.5 µmol) in DMSO (0.1 mL) was added. A THPTA-Cu(II) complex was prepared by mixing tris(3-hydroxypropyltriazolylmethyl)amine (0.5 eq., 0.1 M in DMF, 37.5 µL, 3.75 µmol) and copper sulfate pentahydrate (0.5 eq., 0.1 M in H₂O, 37.5 µL, 3.75 µmol) and incubating at 21°C for 1 minute. The THPTA-Cu(II) complex (0.5 eq., 0.05 M in DMF/H₂O 50/50, 75 µL, 3.75 µmol) was added to the reaction mixture followed by sodium ascorbate (2.5 eq., 0.5 M in H₂O, 37.5 µL, 18.75 µmol). The resulting mixture was incubated at 21°C for 15 min and purified by preparative HPLC (method 1) to yield **S7** (7.44 mg, 4.58 µmol, 61 %) as a white solid.
**HPLC (Method 2) RT:** 3.95 min
**MS (Method 3) *m*/*z*:** 813.9 [M+2H]²⁺/2

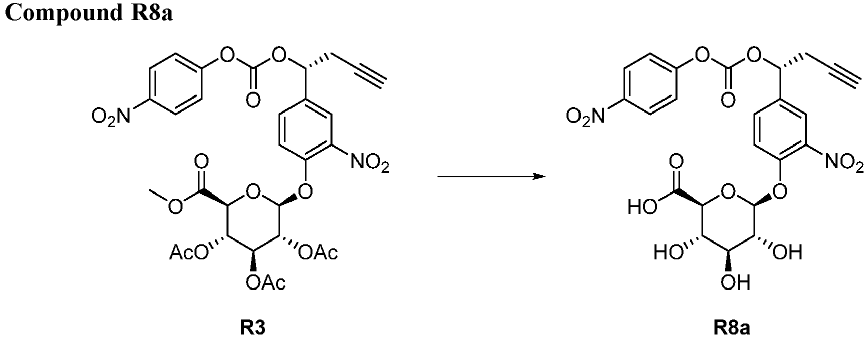

To a solution of **R3** (1 eq., 317 mg, 0.46 mmol) in acetonitrile (2 mL) was added concentrated HCl (52.2 eq., 2 mL, 24 mmol) and the mixture was stirred at 37°C for 16h. To the resulting mixture was added EtOAc (8 mL), followed by the addition of water (8 mL). The organic layer was separated and the aqueous layer was extracted three times with EtOAc. Combined organic layer was dried over MgSO₄ and concentrated under vacuum to yield the crude **R8a** (250 mg, 0.46 mmol, 99%), which was used in the next step without further purification.

**MS (Method 3) *m*/*z*:** 549.1 [M+H]⁺

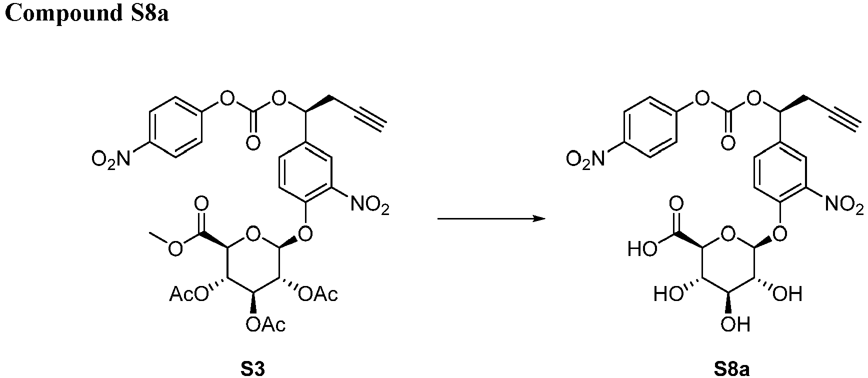

To a solution of **S3** (1 eq., 317 mg, 0.46 mmol) in acetonitrile (2 mL) was added concentrated HCl (52.2 eq., 2 mL, 24 mmol) and the mixture was stirred at 37°C for 16h. To the resulting mixture was added EtOAc (8 mL), followed by the addition of water (8 mL). The organic layer was separated and the aqueous layer was extracted three times with EtOAc. Combined organic layer was dried over MgSO₄ and concentrated under vacuum to yield the crude **S8a** (247 mg, 0.45 mmol, 98%), which was used in the next step without further purification.

**MS (Method 3) *m*/*z*:** 549.1 [M+H]⁺

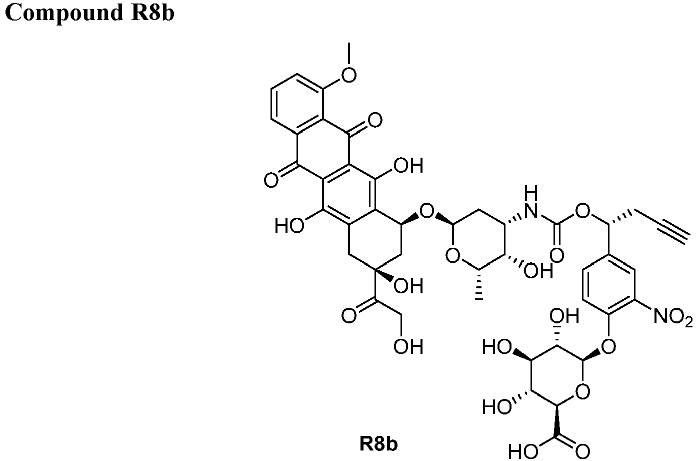

To a solution of doxorubicin hydrochloride (1 eq., 10 mg, 0.017 mmol) in DMF (0.35 mL) was added a solution of TEA (2 eq., 0.35 mL, 0.1 M in DMF, 0.035 mmol), followed by the addition **R8a** (1 eq., 9.5 mg, 0.017 mmol). The resulting mixture was stirred at 25°C for 16h and purified by preparative HPLC (method 1) to yield **R8b** (12.2 mg, 0.013 mmol, 74%) as a red solid.

**MS (Method 3) *m*/*z*:** 953.3 [M+H]⁺

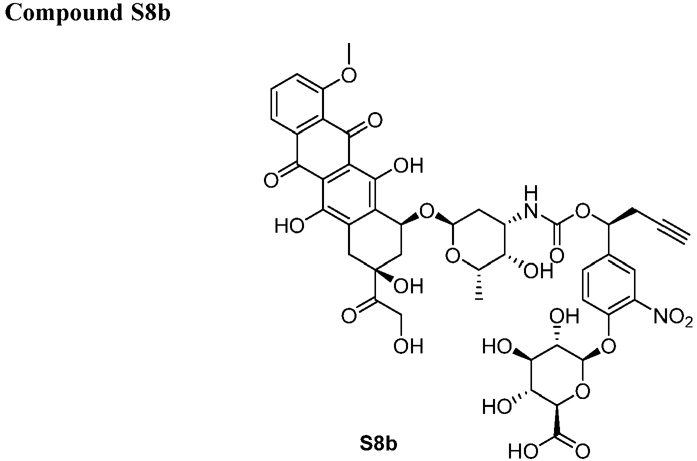

To a solution of doxorubicin hydrochloride (1 eq., 10 mg, 0.017 mmol) in DMF (0.35 mL) was added a solution of TEA (2 eq., 0.35 mL, 0.1 M in DMF, 0.035 mmol), followed by the addition **S8a** (1 eq., 9.5 mg, 0.017 mmol). The resulting mixture was stirred at 25°C for 16h and purified by preparative HPLC (method 1) to yield **S8b** (13.2 mg, 0.014 mmol, 80%) as a red solid.

**MS (Method 3) *m*/*z*:** 953.3 [M+H]⁺

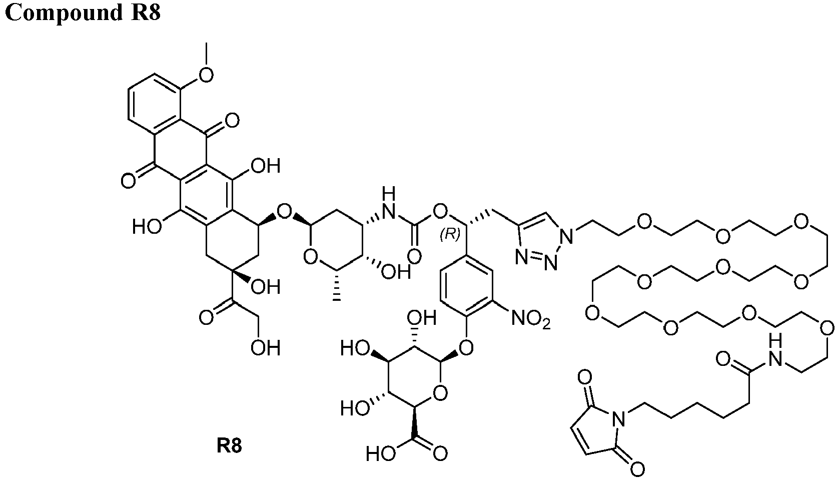

To the solution of 32-azido-3,6,9,12,15,18,21,24,27,30-decaoxadotriacontan-1-amine (1.5 eq., 11.82 mg, 22.45 µmol) in dry DMF (0.2 mL) was added a solution of 2,5-dioxopyrrolidin-1-yl 6-(2,5-dioxo-2,5-dihydro-1H-pyrrol-1-yl)hexanoate (1.5 eq., 6.92 mg, 22.45 µmol) in dry DMF (0.2 mL). The mixture was stirred at 21°C for 1 hour and then **R8b** (1 eq., 12 mg, 14.97 µmol) in DMSO (0.2 mL) was added. A THPTA-Cu(II) complex was prepared by mixing tris(3-hydroxypropyltriazolylmethyl)amine (1 eq., 0.1 M in DMF, 150 µL, 14.97 µmol) and copper sulfate pentahydrate (1 eq., 0.1 M in H₂O, 150 µL, 14.97 µmol) and incubating at 21°C for 1 minute. The THPTA-Cu(II) complex (1 eq., 0.05 M in DMF/H₂O 50/50, 300 µL, 14.97 µmol) was added to the reaction mixture followed by sodium ascorbate (2 eq., 0.5 M in H₂O, 60 µL, 29.94 µmol). The resulting mixture was incubated at 21°C for 15 min and purified by preparative HPLC (method 1) to yield **R8** (14.35 mg, 9.43 µmol, 63 %) as a red solid.
**HPLC (Method 2) RT:** 3.70 min
**MS (Method 3) *m*/*z*:** 836.9 [M+2H]²⁺/2

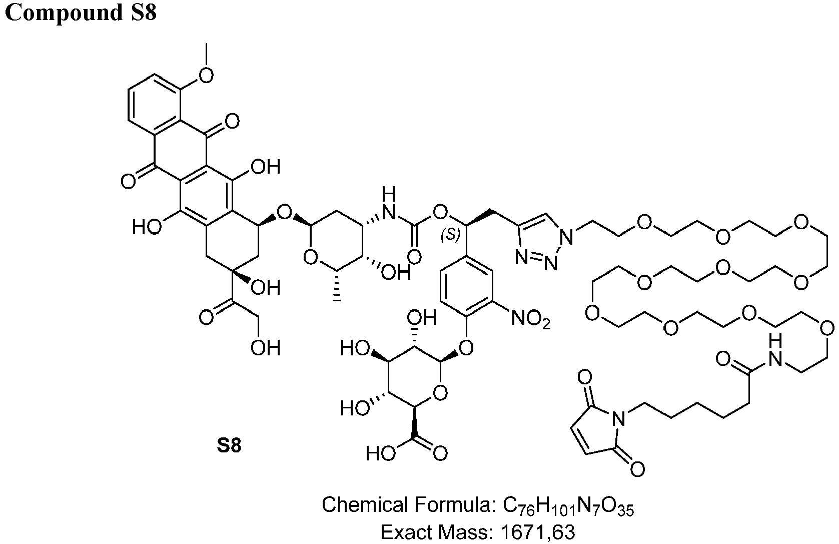

To the solution of 32-azido-3,6,9,12,15,18,21,24,27,30-decaoxadotriacontan-1-amine (1.5 eq., 11.82 mg, 22.45 µmol) in dry DMF (0.2 mL) was added a solution of 2,5-dioxopyrrolidin-1-yl 6-(2,5-dioxo-2,5-dihydro-1H-pyrrol-1-yl)hexanoate (1.5 eq., 6.92 mg, 22.45 µmol) in dry DMF (0.2 mL). The mixture was stirred at 21°C for 1 hour and then **S8b** (1 eq., 12 mg, 14.97 µmol) in DMSO (0.2 mL) was added. A THPTA-Cu(II) complex was prepared by mixing tris(3-hydroxypropyltriazolylmethyl)amine (1 eq., 0.1 M in DMF, 150 µL, 14.97 µmol) and copper sulfate pentahydrate (1 eq., 0.1 M in H₂O, 150 µL, 14.97 µmol) and incubating at 21°C for 1 minute. The THPTA-Cu(II) complex (1 eq., 0.05 M in DMF/H₂O 50/50, 300 µL, 14.97 µmol) was added to the reaction mixture followed by sodium ascorbate (2 eq., 0.5 M in H₂O, 60 µL, 29.94 µmol). The resulting mixture was incubated at 21°C for 15 min and purified by preparative HPLC (method 1) to yield **S8** (15.5 mg, 10.18 µmol, 68 %) as a red solid.
**HPLC (Method 2) RT:** 3.65 min
**MS (Method 3) *m*/*z*:** 836.9 [M+2H]²⁺/2

### Determination of absolute configuration of R1 through R1a, by X-Ray crystallography

### 1) Preparation of R1a

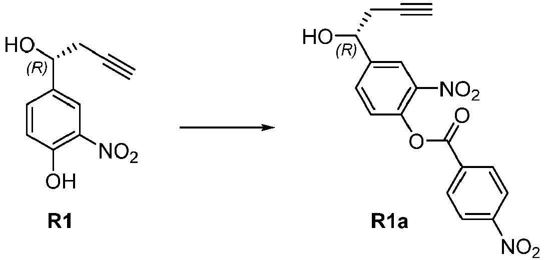

To determine absolute configuration of **R1,** ester **R1a** was synthesized, crystallized and analyzed by X-ray crystallography. To a cooled to 0°C solution of **R1** (1 eq., 111 mg, 0.536 mmol) and triethylamine (2 eq., 108 mg, 0.149 mL, 1.07 mmol) in THF (3.61 mL) was added dropwise a solution of 4- nitrobenzoyl chloride (1.5 eq., 149 mg, 0.804 mmol) in THF (3.61 mL). The resulting solution was warmed to room temperature and left for 30 minutes. Ethyl acetate (22 mL) was added followed by the addition of 7 mL of water. The organic layer was separated and washed with saturated NaCl solution. The organic layer was evaporated and the residue was purified by flash chromatography (cyclohexane/EtOAc gradient 0-100% of EtOAc in 20 minutes; 30 column volumes) to afford **R1a** (60.5 mg, 0.17 mmol, 88% yield).

### 2) Determination of absolute configuration of R1a by X-Ray crystallography

**R1a** (60 mg) was dissolved in 3 mL of dichloromethane and diluted with 3 mL of heptane. The mixture was allowed to slowly evaporate over a period of 2 weeks, inducing the formation of **R1a** crystals. The crystals were placed in oil, and a colourless plate single crystal of dimensions 0.50 × 0.40 × 0.18 mm was selected, mounted on a glass fiber and placed in a low-temperature N₂ stream. X-Ray diffraction data collection was carried out on a Bruker APEX II DUO Kappa-CCD diffractometer equipped with an Oxford Cryosystem liquid N₂ device, using Cu-Kα radiation (λ = 1.54178 Å). The crystal-detector distance was 40mm. The cell parameters were determined (APEX3 software) from reflections taken from three sets of 20 frames, each at 10s exposure. The structure was solved using the program SHELXT-2014. The refinement and all further calculations were carried out using SBELXL-2014. The hydrogen atom of one OH group was located from Fourier difference. The other H-atoms were included in calculated positions and treated as riding atoms using SHELXL default parameters. The non-H atoms were refined anisotropically, using weighted full-matrix least-squares on F². A semi-empirical absorption correction was applied using SADABS in APEX3; transmission factors: T_{min/}Tₘₐₓ = 0.6111/0.7528.

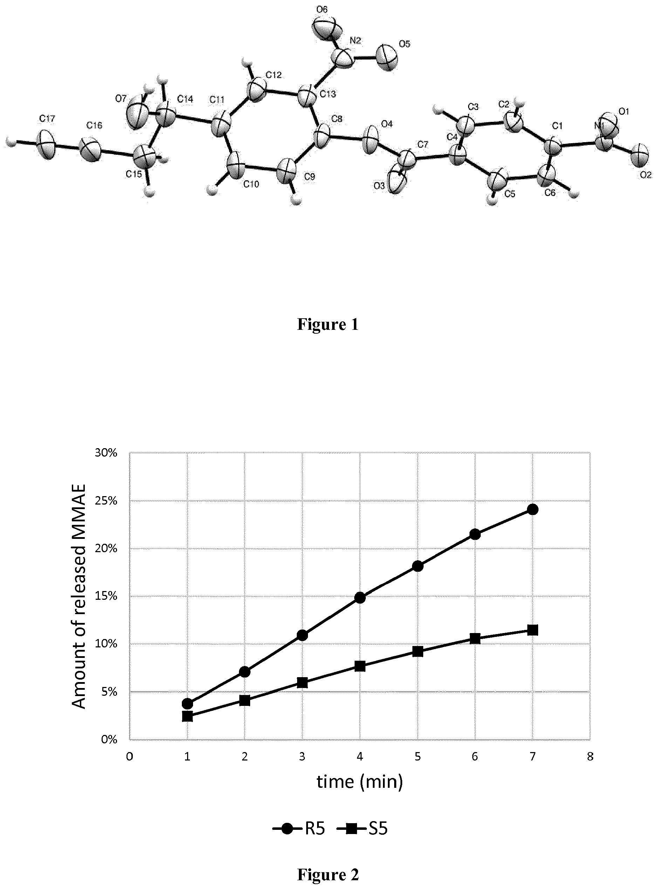

Results of the X-ray crystallography are presented on Figure 1.

### Measurement of the kinetics of β-glucuronidase-induced release of MMAE

- Sample solution of R isomer was prepared by adding 10 µL of **R5** (10 mM in DMSO) to 990 µL of human plasma and incubating at 37°C for 20 min.
- Sample solution of S isomer was prepared by adding 10 µL of **S5** (10 mM in DMSO) to 990 µL of human plasma and incubating at 37°C for 20 min.
- Quenching solution (**Q**) was prepared by adding 50 µL of 1 M aqueous HCl to 1.5 mL of acetonitrile.
- β-glucuronidase solution (**Glu**) was prepared by adding 20 µL of aqueous glycerol solution of β-glucuronidase from *Escherichia coli* (6.5 mg/mL) to 180 µL of H₂O.
- Reference solution (**Ref**) was prepared by adding 10 µL of **MMAE** (10 mM in DMSO) to 990 µL of human plasma and incubating at 37°C for 20 min.

To 1 mL of each isomer and reference solution was added 40.9 uL of **Glu.** The resulting mixtures were incubated at 37°C. 45 µL aliquots of each solution were quenched with 155 µL of **Q** at the following time points: 1 min, 2 min, 3 min, 4 min, 5 min, 6 min and 7 min. Quenched aliquots were centrifuged at 15000 g for 5 min and the supernatant was analyzed by LCMS (Method 3) with Selected ion recording set to 719 Da (corresponding to [MMAE+H]⁺). The amount of released MMAE at each time point was calculated as (sample peak area)/(reference peak area) × 100% (Figure 2).

### General procedure for the measurement of the kinetics of β-glucuronidase-induced release of a cytotoxic drug

- Sample solution of R isomers was prepared by adding 5 µL of compounds **R6, R7** or **R8** (10 mM in DMSO) to 500 µL of a 50/50 mixture of human plasma and potassium phosphate buffer (0.1M, pH 7.0) and incubating at 37°C for 20 min.
- Sample solution of S isomers was prepared by adding 5 µL of compounds **S6, S7** or **S8** (10 mM in DMSO) to 500 µL of a 50/50 mixture of human plasma and potassium phosphate buffer (0.1M, pH 7.0) and incubating at 37°C for 20 min.
- Quenching solution (**Q**) was prepared by adding 50 µL of 1 M aqueous HCl to 1.5 mL of acetonitrile.
- β-glucuronidase solution (**Glu**) was prepared by adding 20 µL of aqueous glycerol solution of β-glucuronidase from *Escherichia coli* (6.5 mg/mL) to 180 µL of H₂O.
- Reference solution (**Ref**) was prepared by adding 5 µL of a corresponding free cytotoxic drug (10 mM in DMSO) to 500 µL of a 50/50 mixture of human plasma and potassium phosphate buffer (0.1M, pH 7.0) and incubating at 37°C for 20 min.

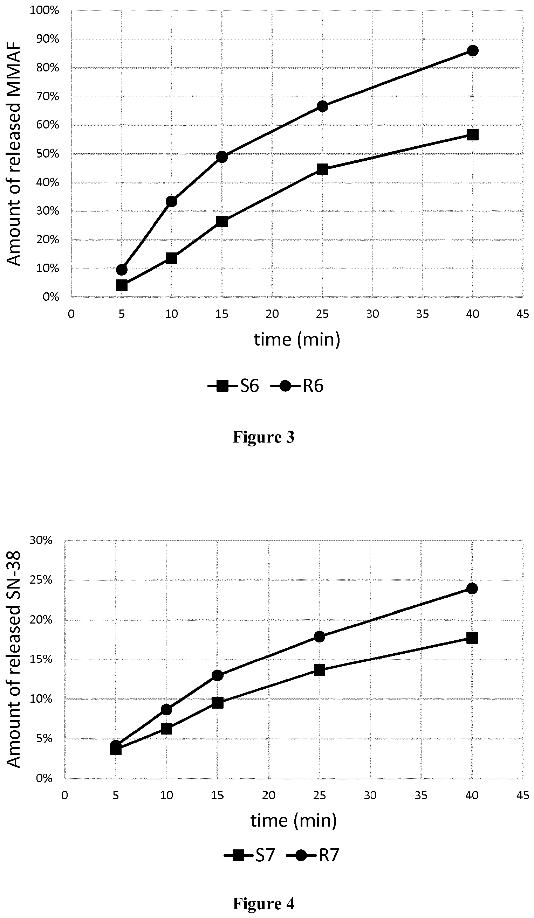

To each sample and reference solution was added 10 uL of **Glu.** The resulting mixtures were incubated at 25°C. 45 µL aliquots of each solution were quenched with 155 µL of **Q** at the precise time points indicated on the graphs (Figures 3-5). Quenched aliquots were centrifuged at 15000 g for 5 min and the supernatant was analyzed by LCMS (Method 3) with Selected ion recording set to [M+H]⁺ where M corresponds to molecular mass of the free cytotoxic agent. The amount of released cytotoxic agent at each time point was calculated as (sample peak area)/(reference peak area) × 100%.

Results are illustrated in Figure 2 to 5:
Figure 2. The kinetics of β-glucuronidase-induced release of MMAE in plasma solutions **of R5** and **S5** demonstrates a significantly faster cleavage of plasma-bound **R5,** compared to **S5** : after 7 minutes of incubation, 24 % of MMAE were released from plasma-bound **R5** and only 12 % from plasma-bound **S5.**
Figure 3. The kinetics of β-glucuronidase-induced release of MMAF in plasma solutions **of R6** and **S6** demonstrates a significantly faster cleavage of plasma-bound **R6,** compared to **S6** : after 40 minutes of incubation, 86 % of MMAF were released from plasma-bound **R6** and only 57 % from plasma-bound **S6.**
Figure 4. The kinetics of β-glucuronidase-induced release of SN-38 in plasma solutions **of R7** and **S7** demonstrates a faster cleavage of plasma-bound **R7,** compared to **S7** : after 40 minutes of incubation, 24 % of SN-38 were released from plasma-bound **R7** and only 18 % from plasma-bound **S7.**

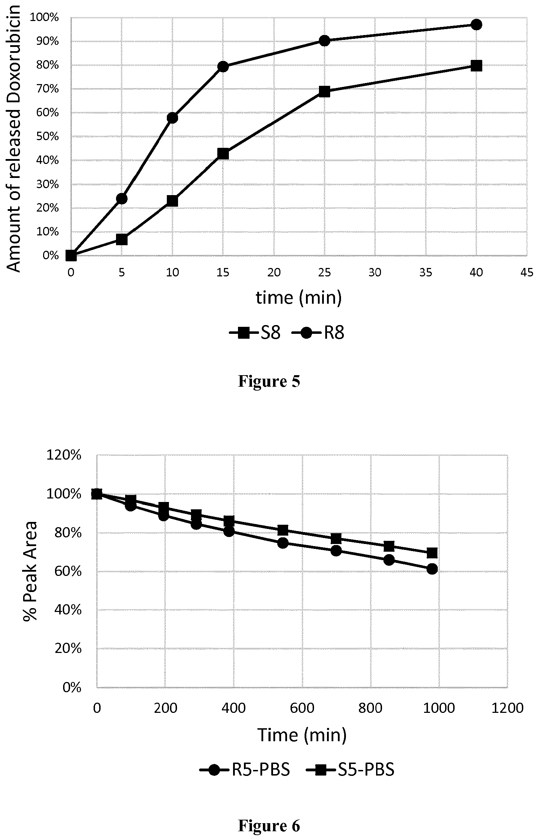

Figure 5. The kinetics of β-glucuronidase-induced release of Doxorubicin in plasma solutions of **R8** and **S8** demonstrates a faster cleavage of plasma-bound **R8,** compared to **S8** : after 40 minutes of incubation, 99 % of Doxorubicin were released from plasma-bound **R8** and 80 % from plasma-bound **S8.**

### Measurement of the stability of R5 and S5 in aqueous buffer at pH 7.4

- Sample solution **R5-PBS** was prepared by adding 20 µL of **R5** (10 mM in DMSO) to 180 µL of phosphate buffered saline (pH 7.4).
- Sample solution **S5-PBS** was prepared by adding 20 µL of **S5** (10 mM in DMSO) to 180 µL of phosphate buffered saline (pH 7.4).
- Samples were incubated at 25°C and analyzed by LCMS (Method 3) with Selected ion recording set to 924 Da (corresponding to [R5+2H]²⁺/2 and [S5+2H]²⁺/2) and to 933 Da (the main impurity being formed corresponding to hydrolyzed products [R5+H₂O+2H]²⁺/2 and [S5+H₂O+2H]²⁺/2). Samples were injected at the following time points: 0 min, 99 min, 195 min, 291 min, 387 min, 543 min, 699 min, 855 min, 980 min. Peak areas of the samples injected at 0 min were considered as reference peak areas.
- % Peak Area was calculated as (924 Da peak area)/((924 Da peak area) + (933 Da peak area)) × 100%.

Results are illustrated in Figure 6. For both samples, a slow hydrolysis of the compound (**R5** or **S5**) was observed at pH 7.4: 30-40 % of the compound is hydrolyzed after 1000 min.

The same experiment was carried out in phosphate buffered saline at pH 6.0. No degradation **of R5** or **S5** was observed in these conditions at any time point.

### Plasma-binding kinetics of R5 and S5

- Quenching solution (**Q**) was prepared by adding 50 µL of 1 M aqueous HCl to 1.5 mL of acetonitrile.
- Sample solution **R-Alb** was prepared by adding 10 µL **of R5** (10 mM in DMSO) to 990 µL of human plasma and incubating at 37°C.
- Sample solution **S-Alb** was prepared by adding 10 µL of **S5** (10 mM in DMSO) to 990 µL of human plasma and incubating at 37°C.
- 45 µL aliquots of each sample solution were added to 155 µL of the quenching solution at the following time-points: 0 min, 1 min, 2 min, 3 min, 4 min, 5 min, 6 min, 7 min.
- Quenched aliquots were centrifuged at 15000 g for 5 min and the supernatant was analyzed by LCMS (Method 3) with Selected ion recording set to 924 Da (corresponding to [R5+2H]²⁺/2 and [S5+2H]²⁺/2). Peak areas of the aliquots quenched at 0 min were considered as reference peak areas.
- The % remaining free drug at each time point was calculated as (sample peak area)/(reference peak area) × 100%.

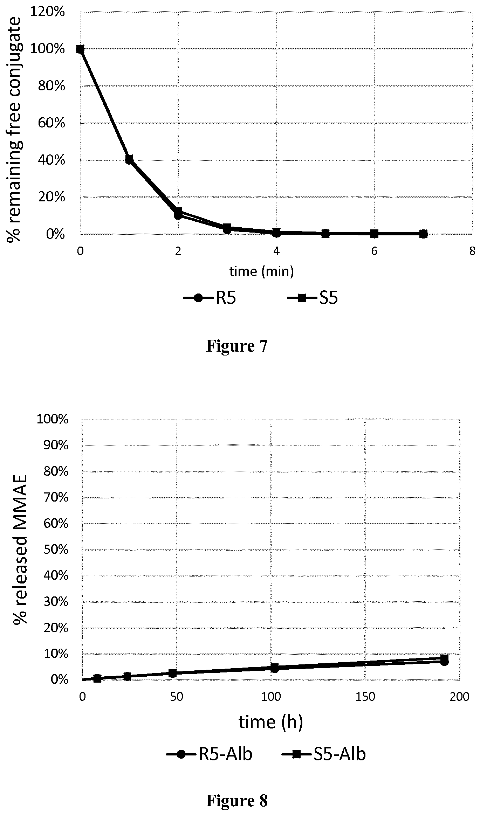

Both compounds **R5** and **S5** undergo rapid binding reaction with serum albumin, resulting in a steep decline of the concentration of their free form, as shown in Figure 7. After 2 minutes of incubation, about 90 % of **S5** or **R5** was bound to serum albumin, and a total conversion was obtained after 4 minutes.

### Plasma stability of albumin-bound R5 and S5

- Sample solution **R5-Alb** was prepared by adding 10 µL of **R5** (10 mM in DMSO) to 990 µL of human plasma.
- Sample solution **S5-Alb** was prepared by adding 10 µL of **S5** (10 mM in DMSO) to 990 µL of human plasma.
- Quenching solution (**Q**) was prepared by adding 50 µL of 1 M aqueous HCl to 1.5 mL of acetonitrile.
- Reference solution (**Ref**) was prepared by adding 10 µL of **MMAE** (10 mM in DMSO) to 990 µL of human plasma.
- Sample solutions and reference solution were incubated at 37°C
- 45 µL aliquots of each sample solution were added to 155 µL of the quenching solution at the following time-points: 0 h, 8 h, 24 h, 48 h, 102 h, 192 h.
- Quenched aliquots were centrifuged at 15000 g for 5 min and the supernatant was analyzed by LCMS (Method 3) with Selected ion recording set to 719 Da (corresponding to [MMAE+H]⁺).
- % Released MMAE at each time point was calculated as (sample peak area)/(reference peak area) × 100%.

As shown in Figure 8, albumin-bound **R5** and **S5** remain stable in plasma, since only trace amounts of the cytotoxic drug (MMAE) were released upon long-term incubation (less than 10% after 192 hours, i.e. 8 days, of incubation).

### Separation tests

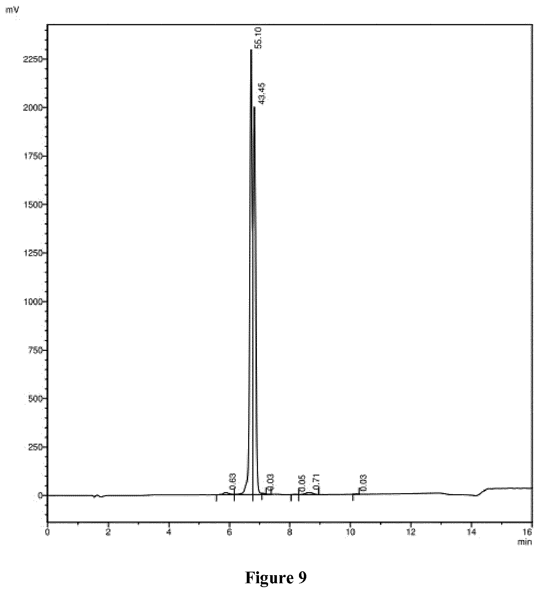

A test aiming at separating compounds R5 and S5 in a 1:1 mixture was carried out by HPLC according to Method 2 but has proven to be unsuccessful: as shown in Figure 9, very close RT's of 6.72 and 6.83 minutes were obtained.

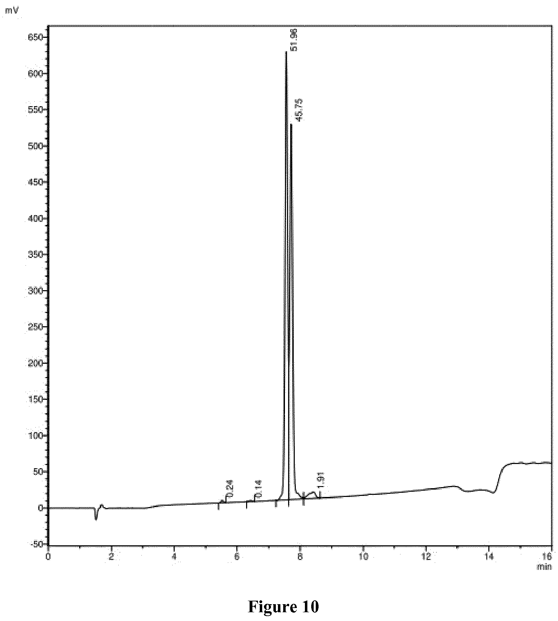

A similar test was carried out with a 1:1 mixture of R4 and S4, but separation has also proven to be unsuccessful, since RT's of 7.56 and 7.72 minutes were obtained (Figure 10).

## Claims

1. A conjugate represented by formula (I) 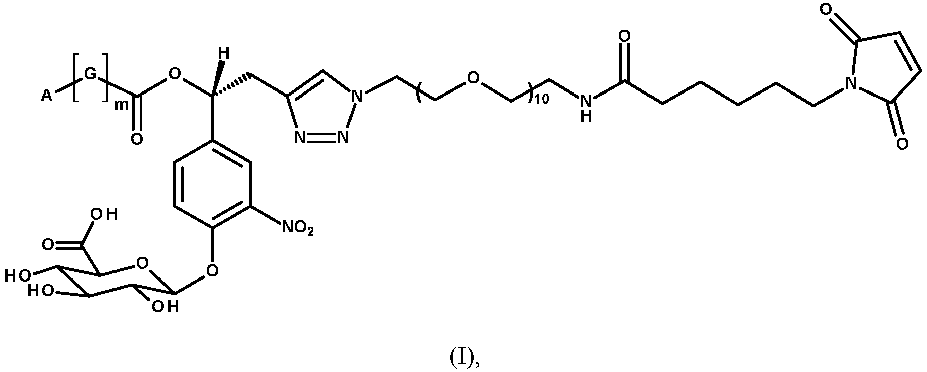 wherein
A represents a radical deriving from a cytotoxic drug,
G is a self-immolative moiety, and
m is 0 or 1,
and pharmaceutically acceptable salts thereof.

2. A conjugate according to claim 1, wherein the cytotoxic drug is selected from anthracyclines, such as doxorubicin, dolastatins such as dolastatin 10, dolastatin 15, auristatin E, auristatin EB (AEB), auristatin EFP (AEFP), monomethyl auristatin F (MMAF), monomethyl auristatin D (MMAD), monomethyl auristatin E (MMAE), 5-benzoylvaleric acid-AE ester (AEVB), and camptothecin analogs chosen from camptothecin, SN-38, topotecan, irinotecan, exatecan, silatecan, cositecan, lurtotecan, gimatecan, belotecan, and rubitecan.

3. A conjugate according to claim 2, wherein the cytotoxic drug is selected from the group consisting of SN-38, doxorubicin, MMAF, MMAE, and MMAD.

4. A conjugate according to any one of claims 1 to 3, wherein G is represented by a formula selected from:

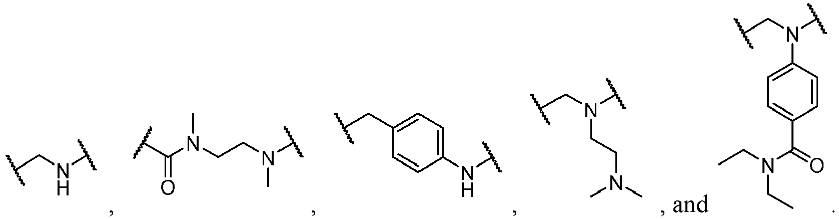

5. A prodrug comprising at least one molecule of the conjugate according to any one of claims 1 to 4, said molecule of the conjugate being linked via a covalent bond to a protein molecule, preferably albumin, or a fragment thereof,
wherein a "prodrug" refers to at least one conjugate covalently linked to a macromolecule.

6. The prodrug according to claim 5, wherein the covalent bond is established with the thiol function of the cysteine in position 34 of the albumin.

7. The prodrug according to claim 5, represented by formula (II), 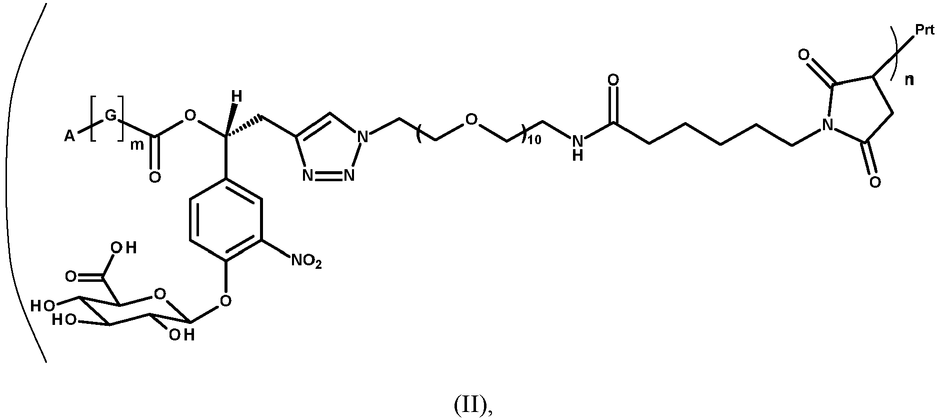 wherein
A represents a radical deriving from a cytotoxic drug,
Prt represents a radical deriving from a protein,
n is comprised between 0.1 and 16, preferably between 0.1 and 8,
G is a self-immolative moiety, and
m is 0 or 1,
and pharmaceutically acceptable salts thereof.

8. The prodrug according to claim 7, wherein Prt represents a radical deriving from albumin and n is 1.

9. A pharmaceutical composition comprising at least an effective amount of at least one conjugate as defined according to any of claims 1 to 4 or at least one prodrug as defined according to any of claims 5 to 8, and a pharmaceutically acceptable carrier.

10. The conjugate according to any of claims 1 to 4, for use in treating a cancer and/or an inflammatory disease.

11. The prodrug according to any of claims 5 to 8, for use in treating a cancer and/or an inflammatory disease.

12. The pharmaceutical composition according to claim 9, for use in treating a cancer and/or an inflammatory disease.

13. A process for preparing a conjugate as defined according to any of claims 1 to 4, comprising the steps of:
a) preparing *rac*-4-(1-hydroxybut-3-yn-1-yl)-2-nitro-phenol from 4-hydroxy-3-nitrobenzaldehyde;
b) separating and isolating (R)-4-(1-hydroxybut-3-yn-1-yl)-2-nitro-phenol, preferably by chiral high-performance liquid chromatography;
c) reacting (R)-4-(1-hydroxybut-3-yn-1-yl)-2-nitro-phenol with a glucuronic acid derivative, such as methyl acetobromo-α-D-glucuronate, under basic conditions;
d) reacting the compound obtained in step (c) with 4-nitrophenyl chloroformate;
e) coupling the compound obtained in step (d) with a cytotoxic drug or with an A-G-H moiety, in which A represents a radical deriving from a cytotoxic drug and G represents a self-immolative moiety;
f) deprotecting the glucuronide moiety of the compound obtained in step (e), preferably under basic conditions; and
g) coupling the compound obtained in step (f) with an azide of formula N₃-(CH₂-CH₂-O)₁₀-(CH₂)₂-NH-(CO)-(CH₂)₅-X, wherein X is a maleimide group.

## Patentansprüche

1. Ein Konjugat repräsentiert durch die Formel (I) 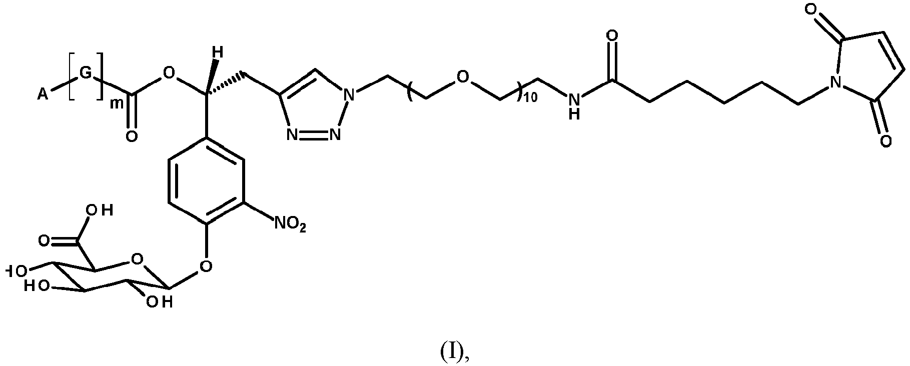 wobei
A ein Radikal abgeleitet von einem zytotoxischen Arzneimittel repräsentiert,
G ein selbst-auflösender Teil ist und
m 0 oder 1 ist,
und pharmazeutisch-verträgliche Salze davon.

2. Ein Konjugat nach Anspruch 1, wobei das zytotoxische Arzneimittel ausgewählt ist aus Anthrazyklinen, wie zum Beispiel Doxorubicin, Dolastatine, wie zum Beispiel Dolastatin 10, Dolastatin 15, Auristatin E, Auristatin EB (AEB), Auristatin EFP (AEFP), Monomethylauristatin F (MMAF), Monomethylauristatin D (MMAD), Monomethylauristatin E (MMAE), 5-Benzoylvaleriansäure-AE Ester (AEVB), und Camptothecin Analoge ausgewählt aus Camptothecin, SN-38, Topotecan, Irinotecan, Exatecan, Silatecan, Cositecan, Lurtotecan, Gimatecan, Belotecan und Rubitecan.

3. Ein Konjugat nach Anspruch 2, wobei das zytotoxische Arzneimittel ausgewählt ist aus der Gruppe bestehend aus SN-38, Doxorubicin, MMAF, MMAE und MMAD.

4. Ein Konjugat nach einem der Ansprüche 1 bis 3, wobei G repräsentiert wird durch eine Formel ausgewählt aus:

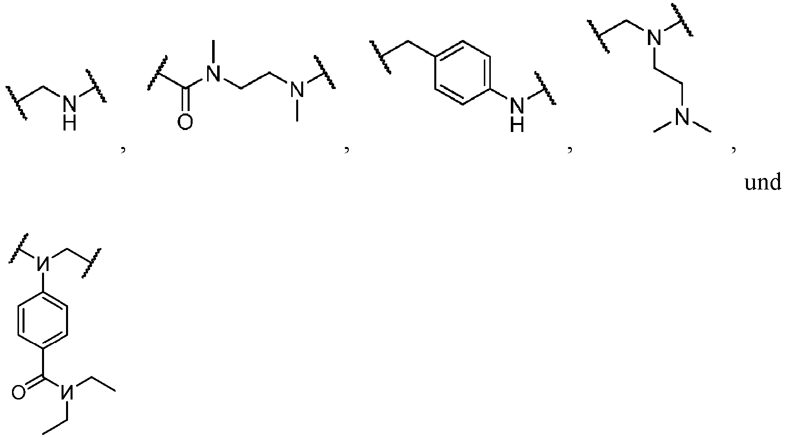

5. Ein Prodrug umfassend mindestens ein Molekül des Konjugats nach einem der Ansprüche 1 bis 4, wobei das Molekül des Konjugats über eine kovalente Bindung an ein Proteinmolekül, bevorzugt Albumin oder ein Fragment davon gebunden ist, wobei sich ein "Prodrug" auf mindestens ein Konjugat, das kovalent an ein Makromolekül gebunden ist, bezieht.

6. Das Prodrug nach Anspruch 5, wobei die kovalente Bindung mit der Thiol-Funktion des Cysteins in Position 34 des Albumins hergestellt wird.

7. Das Prodrug nach Anspruch 5, repräsentiert durch Formel (II), 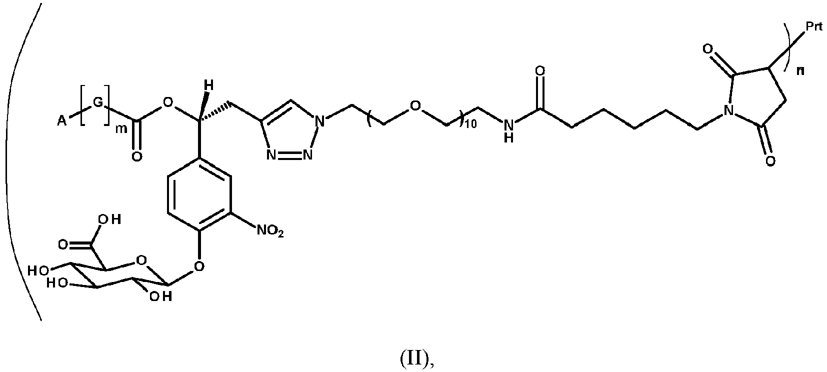 wobei
A ein Radikal abgeleitet von einem zytotoxischen Arzneimittel repräsentiert,
Prt ein Radikal abgeleitet von einem Protein repräsentiert,
n zwischen 0,1 und 16, bevorzugt zwischen 0,1 und 8 liegt,
G ein selbst-auflösender Teil ist und
m 0 oder 1 ist,
und pharmazeutisch-verträgliche Salze davon.

8. Das Prodrug nach Anspruch 7, wobei Prt ein Radikal abgeleitet von Albumin repräsentiert und n 1 ist.

9. Eine pharmazeutische Zusammensetzung umfassend mindestens eine wirksame Menge mindestens eines Konjugats wie nach einem der Ansprüche 1 bis 4 definiert oder mindestens eines Prodrugs wie nach einem der Ansprüche 5 bis 8 definiert und ein pharmazeutisch-verträglicher Träger.

10. Das Konjugat nach einem der Ansprüche 1 bis 4, zur Verwendung in der Behandlung einer Krebserkrankung und/oder einer inflammatorischen Erkrankung.

11. Das Prodrug nach einem der Ansprüche 5 bis 8, zur Verwendung in der Behandlung einer Krebserkrankung und/oder einer inflammatorischen Erkrankung.

12. Die pharmazeutische Zusammensetzung nach Anspruch 9 zur Verwendung in der Behandlung einer Krebserkrankung und/oder einer inflammatorischen Erkrankung.

13. Ein Verfahren zur Herstellung eines Konjugats wie in einem der Ansprüche 1 bis 4 definiert, umfassend die folgenden Schritte:
a) Herstellen von *rac*-4-(1-Hydroxybut-3-yn-1-yl)-2-nitro-phenol aus 4-Hydroxy-3-nitrobenzaldehyd,
b) Trennen und Isolieren von (R)-4-(1-Hydroxybut-3-yn-1-yl)-2-nitro-phenol, bevorzugt mittels chiraler Hochleistungs-Flüssigchromatographie,
c) Reagieren lassen von (R)-4-(1-Hydroxybut-3-yn-1-yl)-2-nitro-phenol mit einem Glucuronsäure-Derivat, wie Methylacetobromo-α-D-glucuronat unter basischen Bedingungen,
d) Reagieren lassen der in Schritt c) erhaltenen Verbindung mit 4-Nitrophenylchlorameisensäureester,
e) Koppeln der in Schritt d) erhaltenen Verbindung mit einem zytotoxischen Arzneimittel oder mit einem A-G-H Teil, in dem A ein Radikal abgeleitet von einem zytotoxischen Arzneimittel repräsentiert und G ein selbst-auflösender Teil ist,
f) Deprotektieren des Glucuronidteils der in Schritt e) erhaltenen Verbindung, bevorzugt unter basischen Bedingungen und
g) Koppeln der in Schritt f) erhaltenen Verbindung mit einem Azid der Formel N₃-(CH₂-CH₂-O)₁₀-(CH₂)₂-NH-(CO)-(CH₂)₅-X, wobei X eine Maleimid-Gruppe ist.

## Revendications

1. Conjugué représenté par la formule (I) 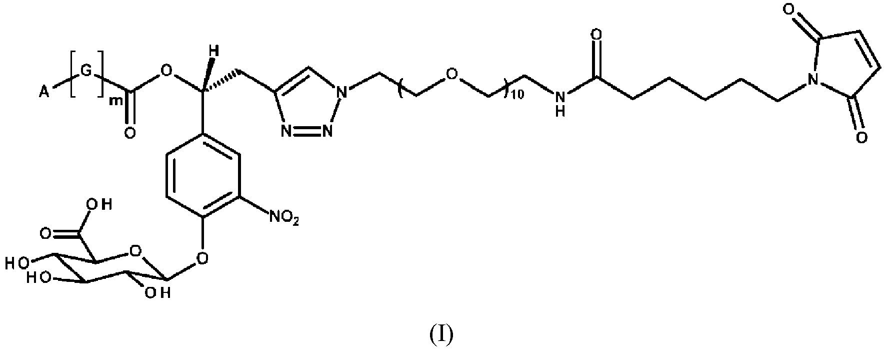 dans laquelle
A représente un radical dérivant d'un médicament cytotoxique,
G est un fragment auto-immolable, et
m vaut 0 ou 1,
et ses sels pharmaceutiquement acceptables.

2. Conjugué selon la revendication 1, dans lequel le médicament cytotoxique est choisi parmi les anthracyclines telles que la doxorubicine, les dolastatines telles que la dolastatine 10, la dolastatine 15, l'auristatine E, l'auristatine EB (AEB), l'auristatine EFP (AEFP), la monométhyl-auristatine F (MMAF), la monométhyl-auristatine D (MMAD), la monométhyl-auristatine E (MMAE), l'ester AE d'acide 5-benzoylvalérique (AEVB), et les analogues de camptothécine choisis parmi la camptothécine, le SN-38, le topotécan, l'irinotécan, l'exatécan, le silatécan, le cositécan, le lurtotécan, le gimatécan, le bélotécan, et le rubitécan.

3. Conjugué selon la revendication 2, dans lequel le médicament cytotoxique est choisi dans le groupe constitué par le SN-38, la doxorubicine, la MMAF, la MMAE, et la MMAD.

4. Conjugué selon l'une quelconque des revendications 1 à 3, dans lequel G est représenté par une formule choisie parmi :

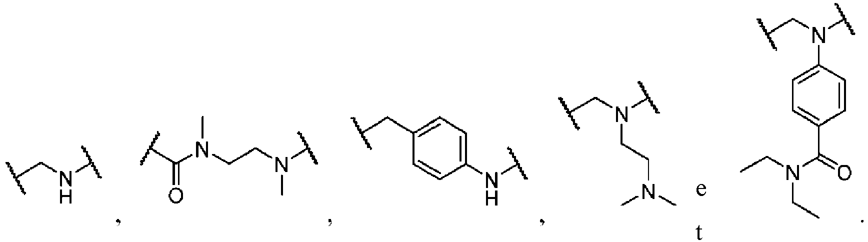

5. Promédicament comprenant au moins une molécule du conjugué selon l'une quelconque des revendications 1 à 4, ladite molécule du conjugué étant liée via une liaison covalente à une molécule de protéine, de préférence l'albumine, ou un fragment de celle-ci,
dans lequel "promédicament" se réfère à au moins un conjugué lié de manière covalente à une macromolécule.

6. Promédicament selon la revendication 5, dans lequel la liaison covalente est établie avec la fonction thiol de la cystéine en position 34 de l'albumine.

7. Promédicament selon la revendication 5, représenté par la formule (II), 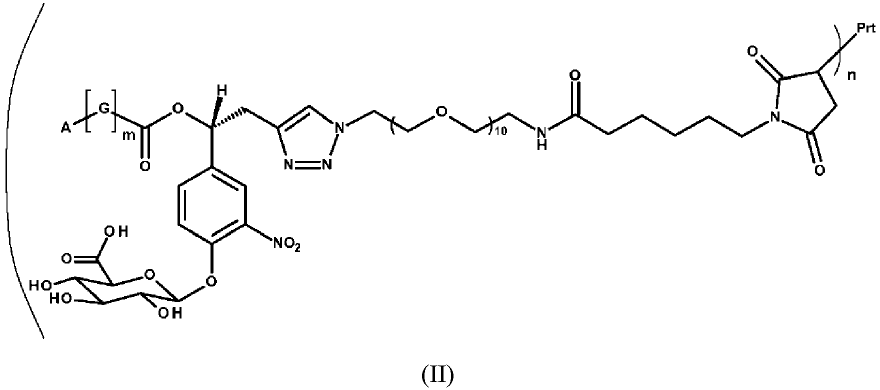 dans laquelle
A représente un radical dérivant d'un médicament cytotoxique,
Prt représente un radical dérivant d'une protéine,
n est compris entre 0,1 et 16, de préférence entre 0,1 et 8,
G est un fragment auto-immolable, et
m vaut 0 ou 1,
et ses sels pharmaceutiquement acceptables.

8. Promédicament selon la revendication 7, dans lequel Prt représente un radical dérivant de l'albumine et n vaut 1.

9. Composition pharmaceutique comprenant au moins une quantité efficace d'au moins un conjugué tel que défini selon l'une quelconque des revendications 1 à 4 ou d'au moins un promédicament tel que défini selon l'une quelconque des revendications 5 à 8, et un véhicule pharmaceutiquement acceptable.

10. Conjugué selon l'une quelconque des revendications 1 à 4, pour une utilisation dans le traitement d'un cancer et/ou d'une maladie inflammatoire.

11. Promédicament selon l'une quelconque des revendications 5 à 8, pour une utilisation dans le traitement d'un cancer et/ou d'une maladie inflammatoire.

12. Composition pharmaceutique selon la revendication 9, pour une utilisation dans le traitement d'un cancer et/ou d'une maladie inflammatoire.

13. Procédé pour préparer un conjugué tel que défini selon l'une quelconque des revendications 1 à 4, comprenant les étapes de :
a) préparation de *rac*-4-(1-hydroxybut-3-yn-1-yl)-2-nitrophénol à partir de 4-hydroxy-3-nitrobenzaldéhyde ;
b) séparation et isolement de (R)-4-(1-hydroxybut-3-yn-1-yl)-2-nitrophénol, de préférence par chromatographie liquide haute performance chirale ;
c) réaction de (R)-4-(1-hydroxybut-3-yn-1-yl)-2-nitrophénol avec un dérivé d'acide glucuronique, tel que l'acétobromo-α-D-glucuronate de méthyle, dans des conditions basiques ;
d) réaction du composé obtenu à l'étape (c) avec du chloroformiate de 4-nitrophényle ;
e) couplage du composé obtenu à l'étape (d) avec un médicament cytotoxique ou avec un fragment A-G-H, dans lequel A représente un radical dérivant d'un médicament cytotoxique et G représente un fragment auto-immolable ;
f) déprotection du fragment glucuronide du composé obtenu à l'étape (e), de préférence dans des conditions basiques ; et
g) couplage du composé obtenu à l'étape (f) avec un azoture de formule N₃-(CH₂-CH₂-O)₁₀-(CH₂)₂-NH-(CO)-(CH₂)₅-X dans laquelle X est un groupe maléimide.
